# EUROPEAN PATENT SPECIFICATION

(11) **EP 3 252 633 B1**
(45) Date of publication and mention of the grant of the patent: **24.06.2020**
(21) Application number: 17171347.2
(22) Date of filing: 16.05.2017
(51) Int. Cl.: G16H 20/30, G16H 20/60, G16H 20/70

(54) **SYSTEM FOR MONITORING PHYSIOLOGICAL ACTIVITY**
SYSTEM ZUR ÜBERWACHUNG VON PHYSIOLOGISCHEN AKTIVITÄTEN
SYSTÈME DE SURVEILLANCE D'UNE ACTIVITÉ PHYSIOLOGIQUE

(30) Priority: 31.05.2016 US 201615169253
(43) Date of publication of application: 06.12.2017
(73) Proprietor: Polar Electro Oy, 90440 Kempele (FI)
(72) Inventor: Posio, Tero, 90440 Kempele (FI); Rönnberg, Lotta, 90440 Kempele (FI); Siekkinen, Petteri, 90440 Kempele (FI)
(74) Representative: Kolster Oy Ab

(56) References cited:
- WO-A1-2014/207294
- WO-A1-2015/069124
- US-A1- 2009 105 560
- US-A1- 2013 158 367
- US-A1- 2013 337 974

## Description

### TECHNICAL FIELD

This invention relates to tracking of physiological activity of a user. More particularly, the present invention relates to tracking physiological activity data of the user and coaching the user based on the physiological activity data.

### BACKGROUND

Measuring physiological activity data, such as training activity data, sleep data and nutrition data, is increasingly becoming more and more popular. In many occasions, measurement and monitoring is limited to only one specific physiological category of a user. However, progressing towards goals may be enhanced using a bit more holistic approach in which different physiological categories of the user are taken into account.

WO 2015069124 discloses a method of monitoring an activity session comprising receiving activity data indicative of at least one activity performed during the activity session, the activity data comprising a plurality of measurements associated to a plurality of parameters monitored during the activity session; a processor comparing at least some of the received measurements associated to at least two of the parameters with at least one set of a plurality of sets of measurements stored on a tangible computer readable medium; and a processor generating a training plan based at least partly on a comparison between the received measurements and the stored measurements.

### BRIEF DESCRIPTION

According to an aspect, there is provided the subject matter of the independent claims.

Some embodiments are defined in the dependent claims.

One or more examples of implementations are set forth in more detail in the accompanying drawings and the description below. Other features will be apparent from the description and drawings, and from the claims.

### BRIEF DESCRIPTION OF DRAWINGS

In the following the invention will be described in greater detail by means of preferred embodiments with reference to the attached drawings, in which
Figure 1 illustrates an example system to which the embodiments of the invention may be applied;
Figure 2 illustrates one or more sensor units that may be used in the system according to an embodiment;
Figure 3 illustrates coaching categories according to an embodiment;
Figures 4, 5A to 5D, and 6A to 6B illustrate some embodiments;
Figures 7A to 7C illustrate coaching categories according to some embodiments;
Figure 8 illustrates a flow diagram according to an embodiment;
Figures 9A to 9C illustrate coaching category -specific instructions according to some embodiments;
Figures 10A, 10B, 10C, and 11 illustrate some embodiments; and
Figure 12 illustrates a block diagram of an apparatus according to an embodiment.

### DETAILED DESCRIPTION OF SOME EMBODIMENTS

The following embodiments are exemplifying. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments. Furthermore, words "comprising" and "including" should be understood as not limiting the described embodiments to consist of only those features that have been mentioned and such embodiments may contain also features/structures that have not been specifically mentioned.

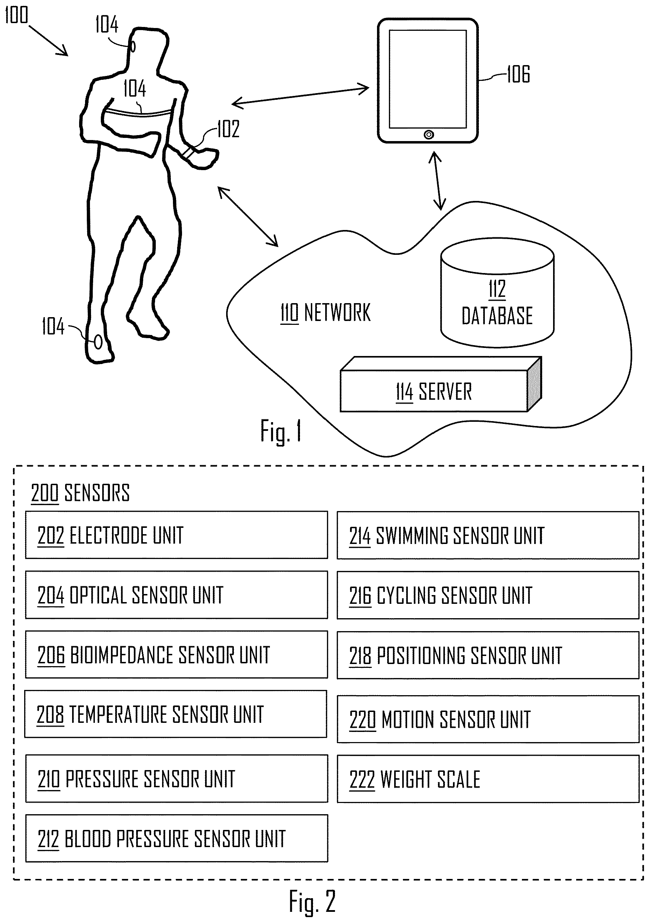

Figure 1 illustrates a system to which embodiments of the invention may be applied. Said system may be used to monitor physical training, activity, and/or inactivity of a user 100 (referred below also just as a/the user(s)). Thus, the embodiments may not be limited to monitoring and/or measuring physical training of the user, and thus said system may be used to monitor physical activity and/or inactivity during the day and/or night (e.g. 24 hours a day). Such may be possible using one or more devices described in relation to Figure 1 and in the embodiments below. In some embodiments, the system may be understood as a system for monitoring well-being of a user or physiological activity or performance of a user. Such monitoring system may comprise, as described, elements for monitoring training. Such monitoring system may comprise elements for monitoring daily activity. Such monitoring system may comprise elements for monitoring inactivity. Such monitoring system may comprise elements for monitoring sleep. Such monitoring system may comprise elements for monitoring stress. Such monitoring system may comprise elements for monitoring nutrition and/or fluid intake. Such monitoring system may comprise elements for monitoring body, such as body composition and/or weight.

Referring to Figure 1, the user 100 may wear a wearable device, such as a wrist device 102. The wrist device 102 may be, for example, a smart watch, a smart device, sports watch, and/or an activity tracking apparatus (e.g. bracelet, arm band, wrist band, mobile phone, glasses). In an embodiment, the wrist device 102 is an activity tracking apparatus or a wearable activity tracking apparatus. This may mean that said apparatus may be worn in other parts of the user 100, such as but not limited to forearm, bicep area, neck, forehead, and/or leg.

In another example, the wearable device may be and/or be comprised in glasses. It may also be possible that a portable electronic device 106 is a wearable device. For example, a phone or smart phone may be carried or holstered such that it actually is a wearable device. However, in some embodiments the wearable device refers to device(s) that are physically connected to a body tissue of the user.

Also, one or more sensor devices 104 worn by the user 100 may be regarded as wearable devices. In some examples, a sensor device may comprise similar functionality as, for example, a smart watch. Also, it may be understood that the wrist device 102 may actually be a sensor comprising a plurality of sensors (e.g. heart rate sensor, motion sensor).

Let us now describe some of the functionalities of the devices of Figure 1. The wrist device 102 may be used to monitor physical activity of the user 100 by using data from internal sensor(s) comprised in the wrist device 102, data from external sensor device(s) 104, and/or data from external services (e.g. from the network 110). It may be possible to receive physical activity-related information from the network 110, as the network may comprise, for example, physical activity-related information of the user 100 and/or some other user(s). Thus, the wrist device 102 may be used to monitor physical activity-related information of the user 100 and/or the other user(s). Naturally, one or more of the external sensor device(s) 104 may be worn by the other user(s), and thus information received from said one or more sensor device(s) 104 may be monitored from the wrist device 102 by the user 100. In a similar way, the portable electronic device (PED) 106 may be used for monitoring purposes. PED 106 may also comprise internal sensor(s) or receive data from other device(s) and/or service(s). For example, the PED 106 may be used to monitor a plurality of sensors worn by the user. Such may be beneficial, for example, for a coach or trainer of a team performing some sports.

It needs to be understood that the wrist device 102 may be used to monitor physical activity of the user 100 and/or to be used as a smart watch configured to enable communication with, for example, a portable electronic device 106, the network 110, and/or some other network, such as a cellular network. Thus, for example, the wrist device 102 may be connected (i.e. wirelessly connected) to the portable electronic device 106, such as a mobile phone, smart phone, tablet and/or computer to name a few. This may enable data transfer between the wrist device 102 and the portable electronic device 106. The data transfer may be based on Bluetooth protocol, for example. Other wireless communication methods, such as, WiFi, Wireless Local Area Network (WLAN) and/or Near Field Communication (NFC), may also be utilized. It is obvious for a skilled person that in order to utilize the techniques, appropriate communication circuitries may need to be used in the devices. For example, a Bluetooth capable circuitry may be needed for Bluetooth communication.

In case of communicating directly with the cellular network, the wrist device 102 and/or the external sensor device(s) 104 may comprise similar communication capabilities as mobile devices, such as 2G, 3G, LTE, LTE-A, 4G and/or 5G communication capabilities. Thus, for example, the wrist device 102 may comprise the communication circuitry capable of operating on said technologies, a Subscriber Identification Module (SIM) and/or a memory comprising a virtual SIM configured to provide a secured identification for the wrist device 102 when operating with the cellular network. The cellular network may be connected to the network 110 and/or comprised in the network 110.

The wrist device 102 may be used to monitor activity and/or inactivity of the user 100. Similarly, the portable electronic device 106 may be used to monitor the activity and/or inactivity of the user 100. Such may require the portable electronic device 106 to acquire physical activity-related data from the wrist device 102, some other wearable device, and/or from external sensor device(s) 104. However, it may be that the portable electronic device 106 determines activity and/or inactivity of the user 100 by utilizing internal sensor(s), such as accelerometer or satellite positioning circuitry. In one example, the PED 106 comprises a camera or a heart activity sensor (e.g. optical heart activity sensor). Such may be used to measure heart activity of the user 100 by placing the sensor appropriately against body tissue of the user 100.

Let us now describe what kind of sensors may be used in the system with reference to Figure 2 showing a plurality of sensors 200 that may be configured to be used in the system of Figure 1. It needs to be noted that many of the sensors 200 may be comprised in two or more devices of the system. For example, there may be a separate motion sensor (e.g. one of the external sensor device(s) 104) and another motion sensor comprised in the wrist device 102.

Referring to Figure 2, the system of Figure 1 may comprise an electrode unit 202 (e.g. electrocardiogram sensor comprising one or more electrodes), an optical sensor unit 204, a bioimpedance sensor unit 206, a temperature sensor unit 208, a pressure sensor unit 210, a blood pressure sensor unit 212, a swimming sensor unit 214, a cycling sensor unit 216, a positioning sensor unit 218, a motion sensor unit 220, and/or weight scale.

In an embodiment, one or more of the sensors 200 are external sensor devices 104. For example, a weight scale 222 may be a separate sensor device. Similarly, a positioning sensor 218 may be a Global Positioning System (GPS) or a GLObal NAvigation Satellite System (GLONASS) device used externally. For example, the electrode unit 202 may be comprised in a heart rate transmitter (e.g. heart rate belt). Similar logic applies to other sensors 200 of Figure 2.

In an embodiment, one or more of the sensors 200 are comprised in the above-described wearable device, such as the wrist device 102. For example, the wrist device 102 may comprise the electrode unit 202, optical sensor unit 204, temperature sensor unit 208, bioimpedance sensor unit 206, pressure sensor unit 210, blood pressure sensor unit 212, positioning sensor unit 218, and/or the motions sensor unit 220.

With reference to Figure 2, sensor unit may imply that said unit comprises one or more of said sensors or, for example, sensor fusion(s). That is, for example, motion sensor unit 220 may comprise one or more motion sensors. Similarly, for example, the cycling sensor unit 216 may comprise one or more sensors (e.g. power sensor(s), motions sensor(s), and/or cadence sensor). Sensor fusion may refer to circuitry that is configured to obtain measurements from two or more sensors and to combine said measurements into measurement data (e.g. motion measurement from two sensors combined into motion measurement data).

In an embodiment, the PED 106 comprises one or more of the sensors 200. For example, the PED 106 may comprise optical sensor unit 204 to measure cardiac activity of the user. This may be possible, for example, if the PED 106 is holstered on an arm holster. Similarly, there may be a temperature sensor unit 208 in the PED 106. It is obvious for a skilled person which of the sensors 200 may be installed in the PED 106. For example, if a normal mobile phone is used, weight scale 222 in said phone would not probably be a good idea. However, if the PED 106 is designed such that it withstand a substantial amount of pressure, it could potentially also be used as a scale. Further, for example, it is obvious for a skilled person that the introduced positioning sensor unit 218 may be used in the PED 106.

As described above, different sensors 200 introduced in Figure 2 may be used with flexibility in the system of Figure 1. The different sensors 200 may, for example, be comprised in one or more devices (e.g. in the wrist device 102, in the external sensor device(s) 104, and/or in the PED 106). Let us then look a bit closer on how different sensors 200 may be used in the system of Figure 1.

With respect to measuring cardiac activity of the user, electrode unit 202, optical sensor unit 204 and/or bioimpedance sensor unit 208 may be used. The one or more sensors used to measure cardiac activity, may be referred to as cardiac activity circuitry or cardiac activity unit, for example. The cardiac activity unit may be used to measure, for example, heart rate, heart beats, Heart Beat Interval (HBI) and/or Heart Rate Variability (HRV). The cardiac activity unit may be shared between devices (e.g. one sensor is an external sensor, one is comprised in the wrist device), or each cardiac activity unit may be comprised in a specific device. For example, sensor fusion may be used to combine the measurements from two or more sensors.

The electrode unit 202 may comprise one or more electrodes configured to measure cardiac activity of the user. Using electrodes may be beneficial in obtaining accurate measurement result. Also it may be convenient to use, for example, an electrode belt under clothes compared to optical measurement from a wrist of the user during winter (e.g. reduces risks of frostbites if the wrist device 102 is used on top of clothes).

The optical sensor unit 204 may comprise, for example, an optical cardiac activity sensor configured to measure cardiac activity of the user. For example, the optical sensor unit 204 may comprise a PPG (photoplethysmography) sensor configured to measure cardiac activity of the user 100. The optical cardiac activity sensor may detect the cardiac activity of the user 100 by optical measurement, which may comprise sending a light beam towards skin of the user and measuring the bounced and/or emitted light from the skin of the user. The light beam may alter when travelling through veins of the user and the alterations may be detected by the optical cardiac activity sensor. By using the detected data, for example, the wrist device 102 may determine cardiac activity of the user, such as heart rate, HRV, and/or HBI.

The biompedance sensor unit 206 may comprise one or more bioimpedance sensors configured to measure, for example, cardiac activity of the user. The bioimpedance sensor unit 206 may be used, however, to measure other things also as explained in further detail below (e.g. stress level). For example, the bioimpedance sensor unit 206 may comprise a cardiac activity sensor and/or skin conductivity sensor configured to measure skin conductance. The skin conductivity sensor may comprise a galvanic skin response (GSR) sensor. The bioimpedance measurement, in general, may be based on transmitting a radio signal into the skin of the user, and observing changes in the radio signal due to impedance changes caused by, for example, blood volume changes. Thus, for example, cardiac activity or stress level of the user may be determined by the wrist device 102 from the data produced by the bioimpedance sensor. It may be possible that only one bioimpedance sensor is used to measure two or more variables (e.g. cardiac activity and stress level).

Further, also other types of biosignal measurement sensors may be embedded into the bioimpedance measurement unit 206. These types include but are not limited to the following: a Laser Doppler-based blood flow sensor, a magnetic blood flow sensor, an Electromechanical Film (EMFi) pulse sensor, a polarization blood flow sensor. Such sensors may be used, for example, to determine cardiac activity of the user.

Again at this point it needs to be noted that the sensor units described in relation to Figure 2 may be comprised in one or more devices. Thus, for example, the bioimpedance sensor unit 206 may be comprised in one or more devices (e.g. one or more external sensors and one or more sensors in the wrist device 102).

Still referring to Figure 2, the temperature sensor unit 208 may comprise one or more sensors for measuring temperature. For example, air temperature, water temperature, and/or core temperature of the user may be measured. One example of measuring core temperature may be to use ear or in-ear sensors for measuring the core temperature. For example, such sensors may be embedded into headphones or similar devices. Also, for example, the wrist device 102 may be used to measure, or at least to estimate, core temperature.

The pressure sensor unit 210 may comprise one or more pressure sensors. In an embodiment, the pressure sensor unit 210 is configured to detect stress or pressure of the user. For example, skin conductivity or sweat measurement may be utilized.

The blood pressure sensor unit 212 may comprise one or more blood pressure sensors configured to measure blood pressure of the user. For example, blood pressure may be measured using an external sensor around user's arm. Such measurement may be known from the field of medical treatment, but is generally unknown in the field of physical activity measurement.

The swimming sensor unit 214 may comprise one or more motion sensors, cadence sensor, and/or speed sensor. In an embodiment, the swimming sensor unit 214 is at least partially comprised in a swimming cap or swimming glasses of a user. For example, a satellite positioning sensor or cardiac activity may be comprised in the swimming cap.

The cycling sensor unit 216 may comprise one or more motions sensors, cadence sensor, speed sensor, and/or power sensor. For example, the swimming cadence may be measured by a cadence sensor comprised in the wrist device 102. The cadence sensor may detect cadence from, for example, movement of a hand or a leg of the user. For example, an external cadence sensor attached to a pedal of a bicycle may be used to measures cycling cadence.

The positioning sensor unit 218 may comprise one or more positioning sensors. Positioning sensors may comprise satellite positioning sensors, such as GPS or GLONASS sensors. The positioning sensors may be used to measure location of the sensor, and consequently the user who is using the sensor. Positioning sensor unit may be used to measure motion (e.g. speed, direction) and/or location of the user, for example.

The motion sensor unit 220 may comprise one or more motion sensors. Each motion sensor may comprise one or more accelerometers, one or more gyroscopes, and/or one or more magnetometers.

In an embodiment, the motion and positioning sensor units 218, 220 are comprised in the same unit. It may be beneficial to use data from both to obtain more accurate data, such as direction, location, and/or movement data, for example. For example, the motion sensor unit 220 may determine movement of the user by determining movement of a hand of the user (e.g. motion sensor in the wrist device 102). Such may also be used, for example, to determine cadence of the user during running or swimming. As described above, the motion sensor unit 220 may also be comprised in some other wearable device and/or in the PED 106. Further, at least one of the external sensor device(s) 104 may comprise the motion sensor unit 220 or part of it. Thus, the motion of the user 100 may be determined in one or more devices of the system.

In an embodiment, the motion sensor unit 220 comprises a compass. The compass may be comprised in the wrist device 102, for example.

In an embodiment, the motion sensor unit 220 comprises a stride sensor. For example, the stride sensor may be attachable to a shoe of the user, and be used to obtain motion measurements. For example, distance travelled may be determined from the data detected by the stride sensor.

In an embodiment, the motion sensor unit 220 comprises an accelerometer and a gyroscope. The motion sensor unit 220 may further comprise sensor fusion software for combining the accelerometer data and gyroscope data so as to provide physical quantities, such as acceleration data, velocity data, or limb trajectory data in a reference coordinate system having orientation defined by a predetermined gyroscope orientation.

In an embodiment, the motion sensor unit 220 comprises a gyroscope and a magnetometer. The motion sensor unit 220 may further comprise sensor fusion software to combine gyroscope data and magnetometer data so as to provide a reference coordinate system for the gyroscope based on the Earth magnetic field measured by the magnetometer. In general, the sensor fusion software described above may combine measurement data acquired from at least two motion sensors such that measurement data acquired from one motion sensor is used to establish the reference coordinate system for the measurement data acquired from at least one other motion sensor. Thus for example, the satellite positioning data may also be utilized in the sensor fusion.

Further, sensor fusion may be used with other sensor units of the sensor 200. For example, sensor fusion may be used with the cardiac activity circuitry in determining the cardiac activity of the user. For example, the sensor fusion software may use one sensor during swimming and another during running. For example, if cardiac activity is measured with two sensors, the system may utilize the data that is currently available.

It also needs to be noted that the sensors 200 of Figure 2 may produce raw measurement data. Also, it may be possible that the sensors 200 process the raw measurement data. For example, the raw measurement data from one or more sensor units may be processed into one or more physiological parameters characterizing physiological state or value(s) of the user. For example, raw measurement data may comprise detected heart beats, the processed physiological parameter may comprise HRV, HBI, and/or heart rate which characterize cardiac activity of the user.

It also needs to be noted that the data obtained by the sensors 200 (e.g. raw measurement data and/or processed data) may be further processed by one or more devices or circuitries of the system of Figure 1. The sensors 200 may transmit data to the wrist device 102, to the network 110 (e.g. database 112, server 114), and/or to the PED 106. Data may be transmitted wirelessly or by using a wired connection. During use, a wireless connection may be beneficial when using external sensors, but not always required (e.g. using headphone sensor or similar with a wired connection). The wrist device 102, the server 114 and/or the PED may process the data obtained from the sensors 200 and process the data into one or more physiological parameters. If a sensor is comprised in a device (e.g. optical sensor in the wrist device 102), it needs to be understood that the transmitting may refer to transferring the data from the sensor to the device or to another circuitry of the device. E.g. from a sensor of a device to a processing circuitry of said device using a connection between the two.

Referring to Figure 1, the external sensor device(s) 104 may, as described, comprise one or more of the sensors 200. The external sensor device(s) 104 may comprise devices such as head sensors, glasses, cardiac activity belts, and team sport monitoring devices, to name a few examples. Each of the devices 104 may comprise one or more of the sensors 200.

For example, the head sensor may be configured to measure cardiac activity and/or skin conductance of the user. The head sensor may be, for example, an ear sensor which may be placed in physical connection with an ear and/or ears of the user. The placement may be similar to placing earplug headphones, for example. Another example may be to use a clip mechanism and/or glue-like material for the physical connection. The head sensor may utilize electrodes, optical measurement and/or bioimpendace measurement for the cardiac activity measurement, for example.

In an embodiment, the ear sensor is an in-ear sensor.

In an embodiment, the external sensor device(s) 104 comprise the ear sensor, such as the in-ear sensor. As described, the ear sensor may be used to measure, for example, cardiac activity of the user 100.

In an embodiment, the head sensor is comprised in glasses or goggles. In such case the head sensor may be comprised in earpiece(s) of the glasses, for example.

In an embodiment, the head sensor is comprised in headphones and/or earphones.

The external sensor device(s) 104 may transmit the sensor data to the wrist device 102, to some other wearable device, to the portable electronic device 106 and/or to a server 114, wherein the server is accessible via a network 110. The wrist device 102, the portable electronic device 106 and/or the server 114 may receive the sensor data. For example, the transmission may be wireless. Data may be stored in the database 112 by the server 114, for example.

Still referring to Figure 1, the external sensor device(s) 104, the wrist device 102, the portable electronic device 106 and/or the server 114 may comprise a communication circuitry, such as wireless communication circuitry, configured to enable data (e.g. sensor data, physical activity data, physiological data) transfer between the external sensor device(s) 104, wrist device 102, portable electronic device 106 and/or the server 114. The communication circuitry may be configured to provide cellular communication capability, Bluetooth communication capability (e.g. Bluetooth Smart), WiFi capability, WLAN capability, Near Field Communication (NFC) capability, and/or LAN capability, to name a few examples. For example, ANT or ANT+ may also be supported. For example, the communication circuitry may support bidirectional wireless communication. For example, the communication circuitry may support unidirectional and/or bidirectional wireless communication. For example, the communication circuitry may support communication on approx. 2.4 GHz radio band or 5kHz band.

Further, the wrist device 102 and/or the portable electronic device 106 may comprise a memory, wherein the memory may be used by the devices to store the data from different sensors 200. Also, processed data (by the sensors 200 or by said devices) may be stored. The server 114 may use a database 112, such as a training database, to store the said data. The database 112 may be accessible via the network 110.

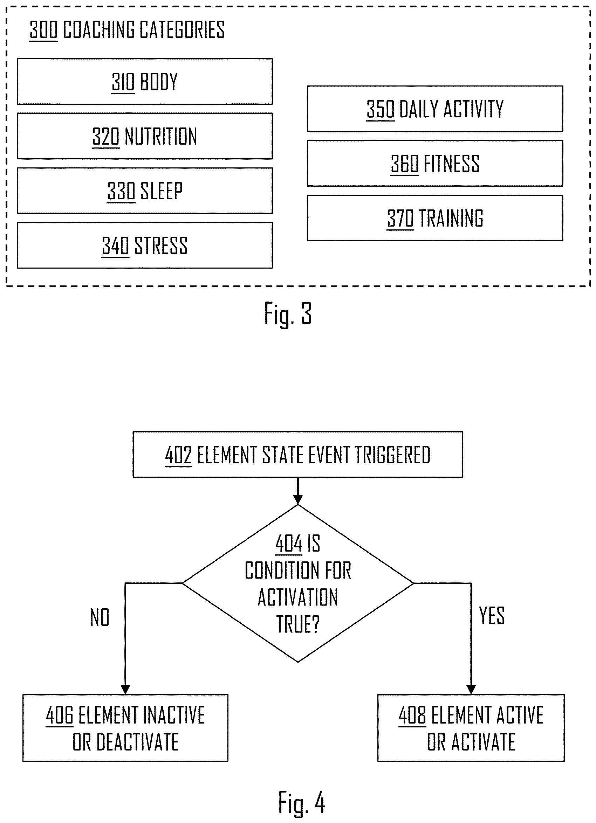

One aspect may be to provide a system configured to assist a user or users in achieving one or more goals or targets. Examples of these goals or targets may comprise weight loss or gain target, getting physically more active, sleep better, stress less, improve fitness, maintain current fitness, get more muscular, and the like. In order to achieve one or more physiological goals, a number of coaching categories are introduced in an embodiment of Figure 3. Referring to Figure 3, the coaching categories 300 may comprise one or more coaching categories. The coaching categories 300 may comprise a body element 310, nutrition element 320, sleep element 330, stress element 340, daily activity element 350, fitness element 360, and/or training element 370. These may be simply referred to as elements or coaching categories. In an embodiment, the coaching categories 310, 320, 330, 340, 350, 360, 370 are all comprised in the coaching categories 300.

The coaching categories 300 may be understood as a part of the system of Figure 1. That is, the coaching categories 300 may be monitored, displayed, processed, and/or determined in said system. Thus, the wrist device 102, the sensor device(s) 104, the PED 106, and/or the network 110 may be used manage the coaching categories 300. For example, goals may be set from a computer using Internet service, and the data may be gathered using the wrist device 102 and/or external sensors 104. For example, progress may be monitored on PED 106, from the server 114, and/or from the wrist device 102. The coaching categories may relate to a specific user or to a group of users: e.g. team coaching categories may be monitored or a target may be set to a family or to a work community.

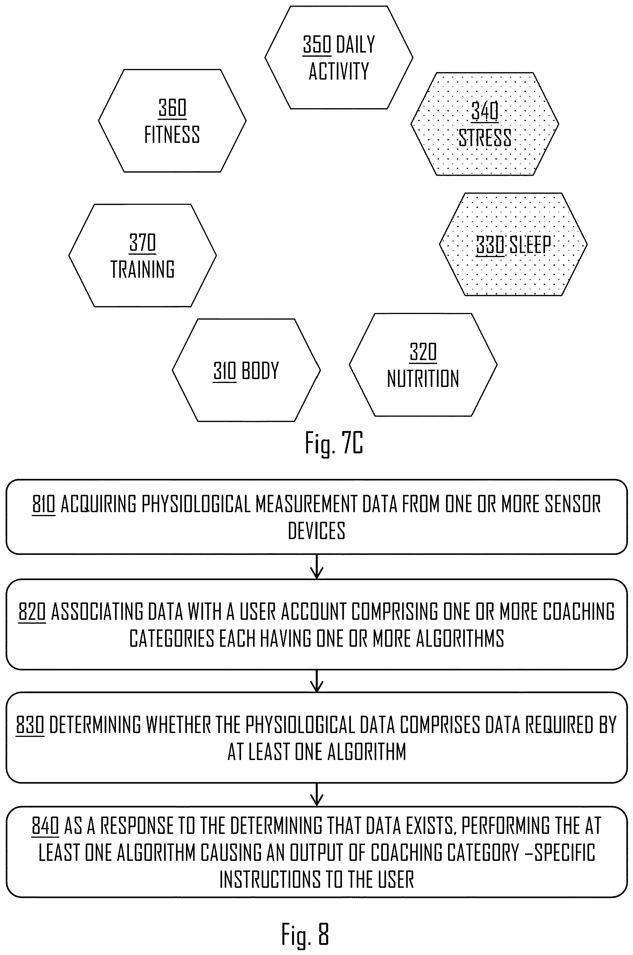

Let us now discuss in more detail what kind of measurements may be performed in relation to the elements 300 (referred also as coaching categories 300, thus each elements 310-370 may be referred to as coaching category also, such as body coaching category 310 or training coaching category 370). Reference is also made to the sensors or sensor units 200 which can be used in performing the measurements. For simplicity reasons, the functions related to managing the coaching categories 300, and performed by the wrist device 102, the external sensor device(s) 104, the PED 106, and/or the server 114, are discussed as being performed by a computing device (or just device) comprising processing means (e.g. circuitries, processors, and/or memories) to carry out the described actions. The computing device may be and/or comprise the apparatus performing the steps of Figure 8.

The body element 310 may utilize and/or comprise data from the electrode unit 202, optical sensor unit 204, bioimpedance sensor unit 206 and/or from the weight scale 222. The measurements and other inputted data in the body element 310 may comprise weight of the user, height of the user, Body Mass Index (BMI) of the user, calorie consumption (e.g. total consumption, consumption during performed training, consumption during daily activity, Basal Metabolic Rate (BMR)), calorie intake, body composition, and/or body composition percent (e.g. muscle, fat, fluids, bones). For example, the body composition may be determined using the bioimpedance measurement by the bioimpedance measurement unit 206.

The nutrition element 320 may utilize data from user input, for example. Such data may comprise meal stamps determining amount, content, and/or time of meals. For example, calorie intake for the body element 310 may be determined from such data. For example, the computing device may acquire user input data about the amount and/or content of food eaten. Further, meal time stamp data may be acquired. Based on said input data, the computing device may determine, for example, calorie intake (e.g. per day, per week).

The sleep element 330 may utilize and/or comprise data from the electrode unit 202, optical sensor unit 204, bioimpedance sensor unit 206, temperature sensor unit 208, pressure sensor unit 210, blood pressure sensor unit 212, and/or from the motion sensor unit 220. The measurements in the sleep element 330 may comprise measuring quality of sleep and/or amount of sleep, for example. Also it may be possible to determine quality of sleep comprising, for example, different stages of sleep, and/or fragmentation of sleep. For example, if the sleep is fragmented, quality may be worse compared with continuous sleep. Further, it may be possible to determine the time when the user has entered sleep (i.e. at what time sleep begins). The motion sensor unit 220 may be used (or data obtained from the motion sensor unit 220), by the computing device, to determine whether the user is sleeping or not if a device comprising the motion sensor unit 220 is worn. From this data, time slept and at which time the sleeping has started may be determined. Further, measuring quality of sleep and/or duration of sleep may comprise determining cardiac activity of the user (e.g. heart rate), respiration rate (e.g. with electrodes 202 or with cardiac activity circuitry 202-206 in general), body temperature (e.g. temperature sensor unit 208) and/or galvanic skin response (e.g. with skin conductance sensor in bioimpedance sensor unit 206).

Still referring to the sleep element 330, the computing device may determine stages of sleep based on the data from the cardiac activity circuitry 202-206 and/or temperature sensor unit 208. For example, temperature may be indicative of different stages of sleep. There may be plurality of stages of sleep which may comprise non-REM sleep and REM sleep. For example, respiration rate (e.g. measured using cardiac activity circuitry 202-206) may indicate the different stages of sleep. Further, HRV may be indicative of different stages of sleep. Thus, the computing device may use data from one or more sensor units 202-208 to determine the stages of sleep. The stages of sleep and time spent in different stages may affect the quality of sleep. For example, at least certain amount of REM sleep may be beneficial to achieve good quality of sleep. On the other hand, restless sleep may be regarded not to be of so good quality.

The stress element 340 may utilize and/or comprise data from the electrode unit 202, optical sensor unit 204, bioimpedance sensor unit 206, temperature sensor unit 208, pressure sensor unit 210, blood pressure sensor unit 212, and/or from the motion sensor unit 220. Measuring stress may comprise determining different physiological values which may be indicative of stress level. For example, perspiration and/or elevated temperature may be signs of increased stress level. Perspiration may be determined by the computing device from measurement data of the bioimpedance unit 206 (e.g. GSR sensor). Temperature may be determined, by the computing device, from measurement data of the temperature sensor unit 208.

Still referring to the stress element 340, stress may have a connection with training too much. Training too much may cause a state, to an exerciser, often referred to as overtraining. In short, this may mean that the exerciser is not recovering from the physical load caused by physical activity, and thus does not develop physically. Stress levels may indicate overtraining. On the other hand, high stress levels may cause overtraining as the time for recovery may decrease (i.e. during high stress recovery may not happen or may be slower). In an embodiment, the computing device initiates at least one test for determining overtraining state and/or stress state of the user. Such test may comprise, for example, an orthostatic test that may show how cardiac activity responds to training and factors such as stress and illness. The orthostatic test may be based on the measurement of heart rate and HRV. Changes in heart rate and HRV may reflect the changes in autonomic regulation of the cardiovascular system. The test may measure beat to beat heart rate, in other words RR intervals. For example, higher stress levels may cause the HRV of the user to be less compared with the HRV in normal stress levels. Thus, during high stress heart beats may become more accurate, therefore decreasing HRV.

In an embodiment, the computing device determines users stress level and/or overtraining level based at least partially on blood pressure measurement. For example, high blood pressure may indicate higher stress levels. Blood pressure measurement, by the blood pressure sensor unit 212, may be used also as supporting some other data. For example, data from GSR sensor and from the blood pressure sensor unit 212 may be combined to achieve more accurate result of the stress levels by the computing device.

Let us now take a look at the coaching categories regarding physical activity and/or inactivity of the user. Said coaching categories may comprise, for example, daily activity element 350, fitness element 360, and/or training element 370.

The daily activity element 350 may utilize and/or comprise data from the electrode unit 202, optical sensor unit 204, bioimpedance sensor unit 206, positioning sensor unit 218, and/or from the motion sensor unit 220. The measurements in the daily activity element 350 may comprise measuring physical activity (e.g. standing, walking, doing home chores, to name a few) and/or measuring inactivity (e.g. sitting, watching TV), for example. The physical activity related to the daily activity element 350 may comprise low level activity which may indicate that moderate and/or vigorous activity performed during physical training may not be part of the daily activity. Low level activity may, in some embodiments, be understood as activity which, at least substantially, does not increase user's physical performance. Thus, for example, very long walking trips may actually be regarded as moderate level activity due to the distance travelled. On the other hand shorter routes by walking may be low level activity (e.g. distances of less than 1 km, 3 km, 5km, or 10 km).

In some embodiments, the daily activity element 350 comprises the low, moderate, and vigorous level activity. Thus, all physical activity performed by the user may be accounted for in the daily activity element 350. Thus, for example, all physical activity may be comprised in the daily activity element 350 whereas only, for example, vigorous level physical activity may be comprised in the training element 370.

Still referring to Figure 3, the daily activity element 350 may relate to metabolic equivalent. This may mean the amount the user is consuming energy (e.g. calories). For example, for 80 kg man basic calorie consumption may be estimated. Further, the physical activity performed by the user may affect the amount of calories are burned. For example, if the user is not performing enough physical activity, the calorie consumption may be lower than the estimated value. On the other hand, performing more physical activity may increase the amount of calories burned.

The activity of the user may be determined from data by the cardiac activity circuitry 202-206, positioning sensor unit 218, and/or the motion sensor unit 220, for example. For example, motion sensor used in a wrist device may indicate the motion of the user. Similarly, cardiac activity (e.g. heart rate) and/or respiratory rate may be used to determine user's physical activity. Inactivity may be determined similarly, but with different value ranges. For example, the computing device may determine that the user has not moved for a predetermined time (e.g. 1 hour), which may cause an inactivity alert. The determination may be based on, for example, motion data from the motion sensor unit 220 and/or from the positioning sensor unit 218.

In an embodiment, the daily activity element 350 indicates whether the user is performing enough physical activity. This may comprise determining, by the computing device, whether the user has been inactive or not. This may comprise determining, by the computing device, whether the user has been active or not. For example, if the computing device determines that a number of inactivity alerts is over a threshold, the computing device may determine that the user is not performing enough physical activity. For example, if the computing device determines that a number of inactivity alerts is under a threshold and that the user is performing physical activity over some threshold (e.g. calorie threshold), the computing device may determine that the user is performing enough physical activity.

In an embodiment, the inactivity and the activity determination, by the computing device, are separate. The computing device may indicate both individually or together. That is, the physical daily activity may be indicated separately (e.g. total calories consumed, percentage of calories consumed from daily target, steps taken, steps taken from a daily target), and thus the daily inactivity may be indicated also separately (e.g. as a number of inactivity alerts).

In an embodiment, the daily activity element 350 comprises measurement data only from the motion sensor unit 220. This may save battery of the computing device and/or sensor used for determining the daily activity. Thus, for example, data from training sessions may be excluded from the daily activity element 350.

In an embodiment, the computing device determines, in the daily activity element 350, activity benefit of the user. The activity benefit may comprise text, video and/or audio indicating benefit of daily activities performed to the user. For example, if the user has not gotten any inactivity alerts during the day, the computing device may indicate the activity benefit to the user, wherein the activity benefit comprises positive feedback. For example, if the user has gotten a plurality of inactivity alerts during the day, the computing device may indicate the activity benefit to the user, wherein the activity benefit comprises feedback that urges the user to do better the next day (e.g. take standing brakes during sitting).

In an embodiment, the computing device determines that the user has been inactive (e.g. sitting and/or not moving) for a predetermined time (e.g. 30 min, 1 hour, 2 hours). As a response, the computing device may cause an output of an indication (e.g. alert message or inactivity alert). The computing device may then proceed on determining whether the user performs activity. If the computing

The fitness element 360 may utilize and/or comprise data from the electrode unit 202, optical sensor unit 204, bioimpedance sensor unit 206, positioning sensor unit 218, and/or from the motion sensor unit 220. At this point, it needs to be understood that the coaching categories 300 may utilize user data in the measurements, calculations, and/or determinations. For example, energy consumption may be affected by user's weight, age and sex. Thus, in an embodiment, the user data may comprise user characteristics.

The user characteristics may comprise user's age, body height, body weight, sex (e.g. female, male), and/or physical activity level, to name a few examples. It needs to be noted that, for example, the body weight may be continuously measured in the body element 310 in some embodiments. Also physical activity may first an estimation by the user (e.g. index from 1 to 5, wherein 1 indicates low level of physical activity and 5 indicates professional athlete), and as the user uses the system of Figure 1, the estimation may be adjusted by the computing device and/or by the user.

Referring to Figure 3, the measurements in the fitness element 360 may comprise measuring physical activity and/or physiological test(s). Let us first discuss the physical activity. The physical activity measurements in the fitness element 360 may comprise measuring versatility and/or volume of the physical activity. The volume may be determined, by the computing device, based on measurements, such as time on speed zones, time on power zones, time on heart rate zones, to name a few examples. In general, volume of training may indicate the intensity of the training and how long certain intensity has been sustained. That is, for example, heart rate zones may in total indicate the duration of the physical activity and further also the intensity of the physical activity. Naturally, simple energy consumption of the performed physical activity may indicate volume of the physical activity.

Versatility of physical activity, however, may indicate does the performed physical activity have enough diversity. For example, versatility may be affected by different sports (e.g. running, swimming, cycling, to name a few) and/or by intensity of training. For example, monotonic exercise on one heart rate zone may not be regarded as versatile. On the other hand training sometimes hard and sometimes with less intensity may be regarded to be more versatile. Also, performing different sports (e.g. cardiovascular training and strength training) may increase versatility of the physical activity.

The physiological test(s) may comprise one or more tests performed during training, using data obtained during training, and/or between training sessions. Said tests may be indicative of fitness development of the user. For example, Running Index may be obtained, by the computing device, based on measurement during a training session (e.g. during a jogging session). The Running Index may indicate how the user is developing with his/her physical condition. Running Index may be just one example of tests which may be performed, by the computing device, during training and/or based on data obtained during training.

On the other hand, said tests may comprise tests which may be performed as tests. This may mean that the user initiates a testing process using the computing device. This may activate one or more of the sensors 200 for the test. Results may be used to determine development of the user. One example of a fitness test, performed as a test, may be the VO2MAX test in which the user's maximum oxygen uptake may be tested. VO2MAX test may require data, for example, from the cardiac activity circuitry 202-206.

The training element 370 may utilize and/or comprise data from the electrode unit 202, optical sensor unit 204, bioimpedance sensor unit 206, the motion sensor unit 220, the temperature sensor unit 208, swimming sensor unit 214, cycling sensor unit 216, and/or from the positioning sensor unit 218. The training element 370 and the fitness element 360 may be more or less connected with each other. However, output of the training element 370 may be seen as one input to the fitness element 360. Thus, in the training element 370 training volume and/or versatility may be measured. Training volume and versatility may be the same or subparts of the physical activity volume and versatility described in relation to the fitness element 360.

Further, as described above, the moderate and vigorous physical activities may relate to the training and/or fitness elements 370, 360, whereas the low level physical activity may relate to the daily activity element 350. However, training and/or fitness elements 370, 360 may also contribute towards the daily activity goal. That is, a performed training session may increase performed daily activity.

In the training element 370, the sensors 200 may be used with versatility to monitor different physical training sessions performed by the user. Physical training may comprise, for example, running, cycling, climbing, swimming, skiing, playing football, playing American football, playing rugby, and the like. In some embodiments, the physical training session starts by the computing device detecting a user input (e.g. start training). Then the computing device may activate one or more sensors 200. These may include internal and/or external sensors. During the training session data may be obtained from the one or more sensors 200, wherein the data may be further processed into physiological parameter(s). For example, the physiological parameter(s) may comprise travelled distance, heart rate, time on heart rate zones, time on speed zones, calories burnt, to name a few examples.

The training element 370 may comprise a recovery status indicator. Said indicator may indicate whether or not the user should perform further training or to rest. For example, the computing device may determine the recovery status based on performed training sessions during a predetermined time (e.g. last one or two weeks). For example, a training session may cause a certain training load. The recovery status may be determined based on cumulated training loads from training sessions. The more there is training load still affecting the user, the more strained the user may be, and thus the recovery status may so indicate. However, if there is not further training load, the recovery status may slowly start to indicate that the user is well rested. For example, the recovery status may indicate at which time the user is ready to perform next training session.

The training element 370 may be understood as element which comprises one or more criteria that the user needs to fulfil in order to obtain certain benefit, target, and/or motivational output from the computing device. For example, the training element 370 may comprise a training program which the user may try to perform. The computing device may determine whether the user is succeeding in performing the training program, and perform an action depending on the determination.

The coaching categories 300 may be connected to each other. This may mean that they may have an effect to each other. For example, progressing in fitness element may cause progress also in body element 310. Also, the coaching categories 300 may relate to one or more goals, wherein each of said goals may relate to one or more coaching categories 300. It also needs to be noted that all of the coaching categories 300 may necessarily not be required. The one or more goals may comprise motivational goals set by a user.

One aspect may be to utilize the different coaching categories 300 to provide the user with one or more personalized instructions to monitor physiological performance of the user. For example and as explained above, the user may select and/or set, using an interface of the system (e.g. web interface or an application), one or more motivational goals for him/herself. The goals may be physiological goals, such as a goal of getting fitter, a goal of maintaining current fitness, a goal of adjusting weight, or a goal of getting more muscle mass, and the like. Using the different coaching categories 800 may provide an enhanced way of providing the instructions to the user as the instructions may be coaching category -specific. This may enable the instructions to be more precise for different areas of physiological performance or general well-being.

Figure 8 illustrates a block diagram according to an embodiment. Figure 8 illustrates a method for physiological monitoring of a user is shown. The method utilizes the different coaching categories, such as the coaching categories 300, in order to provide more accurate instructions to the user. For example, the method may be used to obtain one or more physiological goals by the user. Referring to Figure 8, the method may comprise: acquiring, by an apparatus, physiological measurement data of the user, wherein the physiological measurement data is obtained using one or more sensor units (block 810); associating, by the apparatus, the physiological measurement data with an user account of the user, wherein the user account comprises a plurality of coaching categories for the physiological monitoring of the user, each coaching category comprising one or more algorithms configured to be performed by the apparatus and requiring physiological measurement data as an input (block 820); determining, by the apparatus, whether the acquired physiological measurement data comprises data required by at least one of the algorithms (block 830); and as a response to determining that the acquired physiological measurement data comprises the required data for the at least one algorithm, performing, by the apparatus, the at least one algorithm, wherein the performing causes an output of coaching category -specific instructions to the user in each of the coaching categories comprising the at least one performed algorithm (block 840).

The apparatus performing the steps of Figure 8 may be or be comprised in the server 114, for example. In some embodiments, the apparatus may be or be comprised in the wearable device, such as the wrist device 102 or the PED 106. The sensor units may refer to sensor units 200, for example.

The physiological measurement data, referred to in block 810, may comprise physical activity measurement data, sleep measurement data, stress measurement data, nutrition and/or fluid measurement data, body composition measurement data and/or weight measurement data, to name a few examples.

Block 820 refers to a user account of a user. It needs to be understood that the system, such as the system of Figure 1, may comprise a plurality of user accounts each associated with a specific user. Further, one or more of the plurality of user accounts may be associated with a group of users. Thus, also performance of a group may be monitored (e.g. physiological measurement data of a group of users).

Now as explained each of the coaching categories 300 may comprise one or more algorithms. Each algorithm may require physiological measurement data as an input and the required data may differ from algorithm to algorithm. For example, sleep element 330 may comprise one algorithm for calculating amount of sleep from measurement data. Another algorithm may be for calculating quality of sleep form measurement data. Similarly, in body element 310, weight may be calculated from weight data using one algorithm and body composition may be calculated from body composition data using another algorithm. Similar logic applies to other values and indicators used in different coaching categories 300. In another example, one algorithm may be used to calculate energy consumption. In another example, one algorithm may be used to calculate heart rate or heart rate zone of the user. In another example, one algorithm may be used to calculate steps taken during a day. However, it needs to be noted that one algorithm may possibly be used in more than one coaching category. In some embodiments, one algorithm may be used to calculate multiple physiological values or indicators of the user. For example, sleep quality and amount may be calculated with one algorithm.

Thus, the algorithms may be used to calculate physiological values or indicators, described in relation to Figure 3, based on the physiological measurement data. In order to an algorithm to work correctly and in a desired manner, it may require specific measurement data. For example, energy consumption algorithm may require heart activity data and/or motion data of the user. For example, sleep amount or quality algorithm may require that the user has worn measurement device during sleeping so that appropriate data may be measured.

As the different algorithm(s) are performed, different physiological parameters, values and/or indicators of the user may be obtained. Accordingly, these parameters, values, and/or indicators may be used to provide the coaching category -specific instructions to the user. This may enable the user to know how to improve his/her performance in each category for which the instructions are provided.

In an embodiment, the coaching category -specific instructions are outputted on a portable device of the user. In an embodiment, the coaching category -specific instructions are transmitted over a network to a portable user device associated with the user account. For example, the instructions may be transmitted by the server 114 over the network 110 to the wrist unit 102 or to the PED 106.

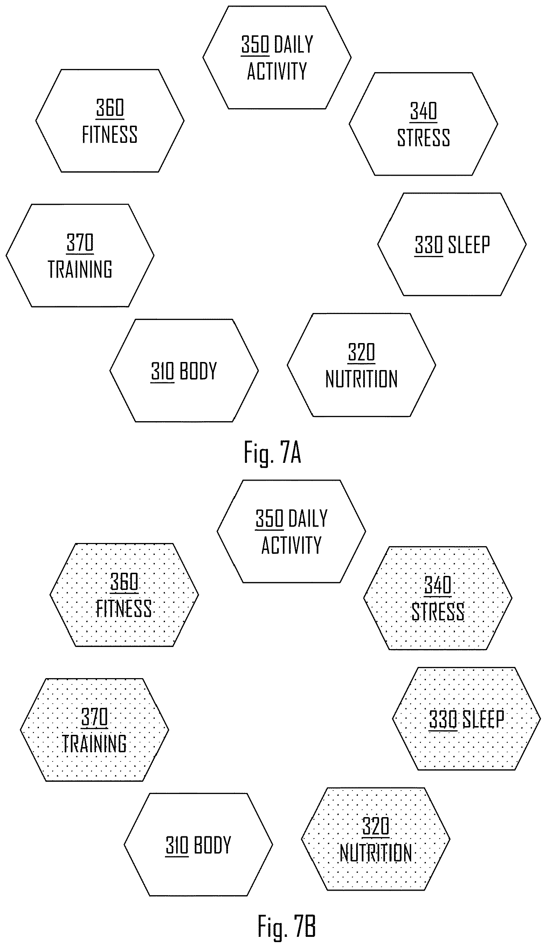

Figures 7A to 7C illustrate some embodiments. Referring to Figures 7A to 7C, a state of the coaching categories 300 may be active or inactive. This may mean, for example, that only for active coaching categories 300 the coaching category -specific instructions are outputted. In an embodiment, only algorithms of active coaching categories 300 are configured to be performed.

Referring to Figure 7A, the state of each of the displayed coaching categories 300 may be active.

Referring to Figure 7B, the state of daily activity element 350 and body element 310 may be active, whereas other coaching categories 300 may be inactive.

Referring to Figure 7C, the state of sleep and stress elements 330, 340 may be inactive, whereas other coaching categories 300 may be active.

In an embodiment, the computing device is configured to activate or deactivate a coaching category according to a user input. For example, the user may select that fitness element 360 should be active and that training element 370 should be inactive.

In an embodiment, the computing device is configured to activate a coaching category if the acquired physiological measurement data comprises data required by at least one algorithm of said coaching category. That is, a coaching category comprising to be performed or performed algorithm may be activated. For example, performing of an algorithm of a certain coaching category may activate said coaching category.

In an embodiment, the computing device is configured to activate a coaching category if the computing device acquires physiological data comprises data required by at least one algorithm of said coaching category. For example, the physiological data may comprise weight and height inputted by the user.

In an embodiment, the computing device is configured to determine a predetermined time period; and to deactivate an active coaching category if none of the at least one algorithms of said active coaching category have been performed during the predetermined time period. That is, a coaching category may be deactivated if none of the algorithms in that category is performed during the predetermined time. This enables the instructions outputted to the user to be as relevant as possible. For example, the user could be annoyed if the computing device would continue to provide training element 370 instructions to the user if the user has only once performed a running exercise. The predetermined time could be, for example, a day, a week, or a month. Also, the predetermined time may be differ net for different coaching categories 300.

In an embodiment, the computing device (e.g. the apparatus of Figure 8) is configured to determine whether or not a coaching category is active and to output instructions only in active coaching categories. That is, in some embodiments, an algorithm of an inactive coaching category may be performed, but this may not cause outputting of instructions in said inactive coaching category.

Figure 4 illustrates an embodiment. Referring to Figure 4, a coaching category 300 activation and deactivation may be illustrated. In block 402, the computing device may detect a coaching category 300 state event associated with a specific coaching category. This may be triggered by user input or by acquiring physiological data of the user. In block 404, the computing device may determine whether the condition for activation or deactivation is true. For example, if the user has selected the specific category to be deactivated, the computing device may proceed to block 406 in which the specific coaching category may be deactivated or maintained inactive. For example, if the user has selected the specific category to be activated, the computing device may proceed to block 408 in which the specific coaching category may be activated or maintained active. The condition in block 404 may comprise, for example, the user input or determining whether or not the acquired physiological data comprises data associated with that specific coaching category. For example, if the acquired data comprises data associated with the specific coaching category (e.g. data required by an algorithm in the specific coaching category), the specific coaching category may be activated or maintained active. Deactivation may also happen if data for the specific coaching category is not received for a predetermined time.

It needs to be noted that in some embodiments, the computing device, such as the apparatus performing the steps of Figure 8 or the server 114, obtains one or more physiological goals of the user. This is also briefly discussed above. Thus, the coaching category -specific instructions may comprise an activity plan for reaching the one or more physiological goals. It needs to be noted that the activity plan may be specific for each coaching category that is active, for example. Thus, for inactive coaching categories, the activity plan may not be outputted.

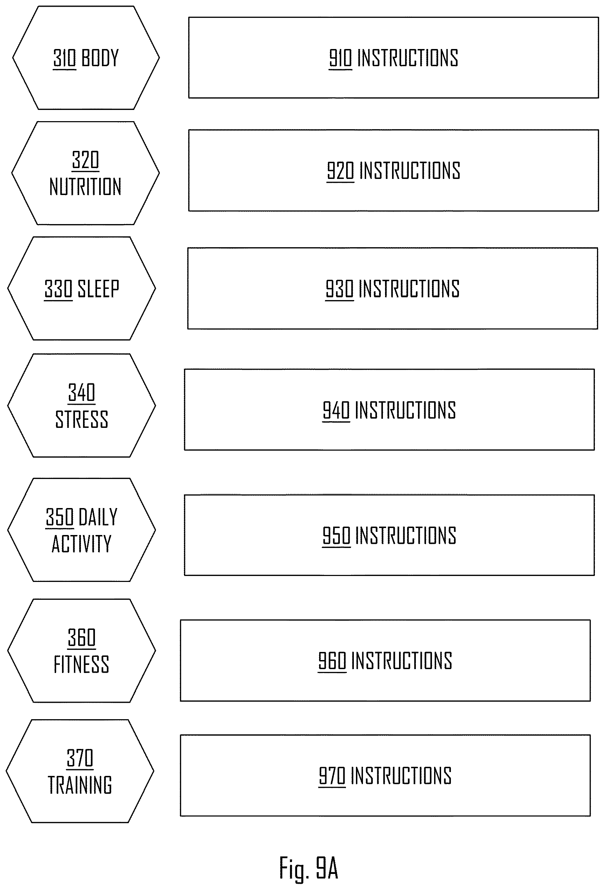

Figures 9A to 9C illustrate some embodiments in which the coaching category -specific instructions 910 - 970 may be shown. Referring to Figures 9A to 9C, the instructions 910 may be associated with the body element 310, the instructions 920 may be associated with the nutrition element 330, the instructions 930 may be associated with the sleep element 330, the instructions 940 may be associated with the stress element 340, the instructions 950 may be associated with the daily activity element 340, the instructions 960 may be associated with the fitness element 360, and/or the instructions 970 may be associated with the training element 370.

Referring to Figure 9A, each of the instructions 910 - 970 may be outputted as each of the coaching categories 300 are active. The outputting may happen regularly or at least when at least one algorithm of each category 300 is performed.

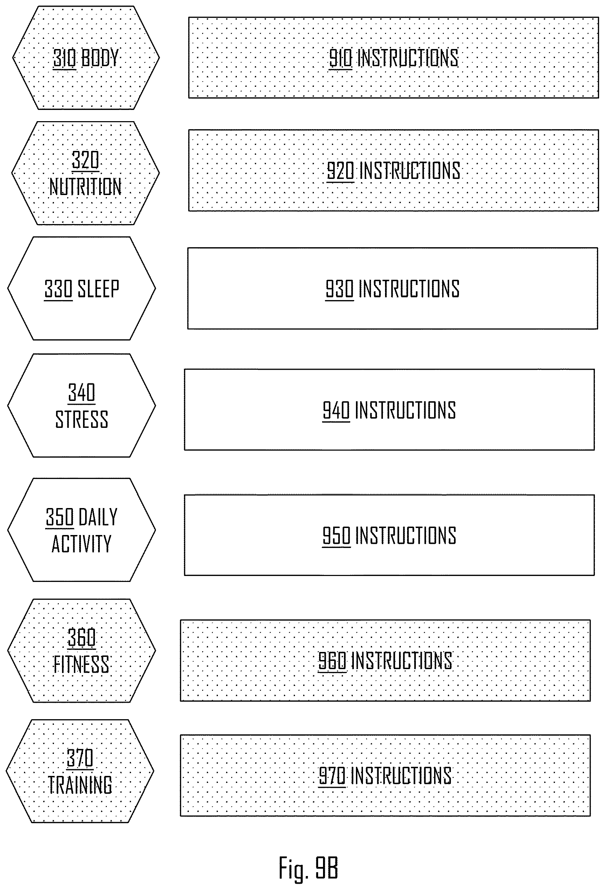

Referring to Figure 9B, instructions 910, 920, 960, 970 may not be outputted as the coaching categories associated with them are inactive and/or they don't comprise algorithms that are performed.

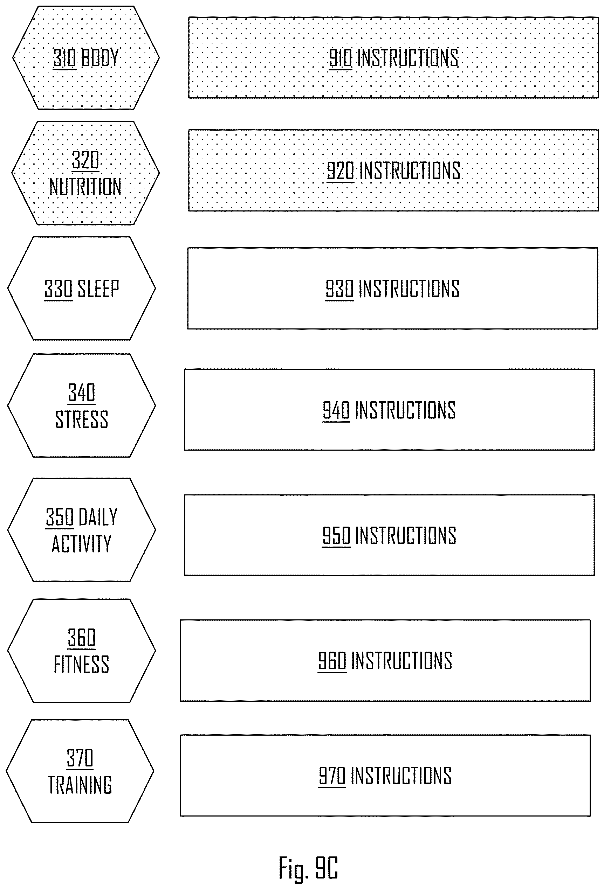

Referring to Figure 9C, the computing may acquire further physiological measurement data compared to the situation of Figure 9B, and thus cause the outputting of instructions 960 and 970. This may also mean, at least in some embodiments, that the respective coaching categories 360, 370 are activated.

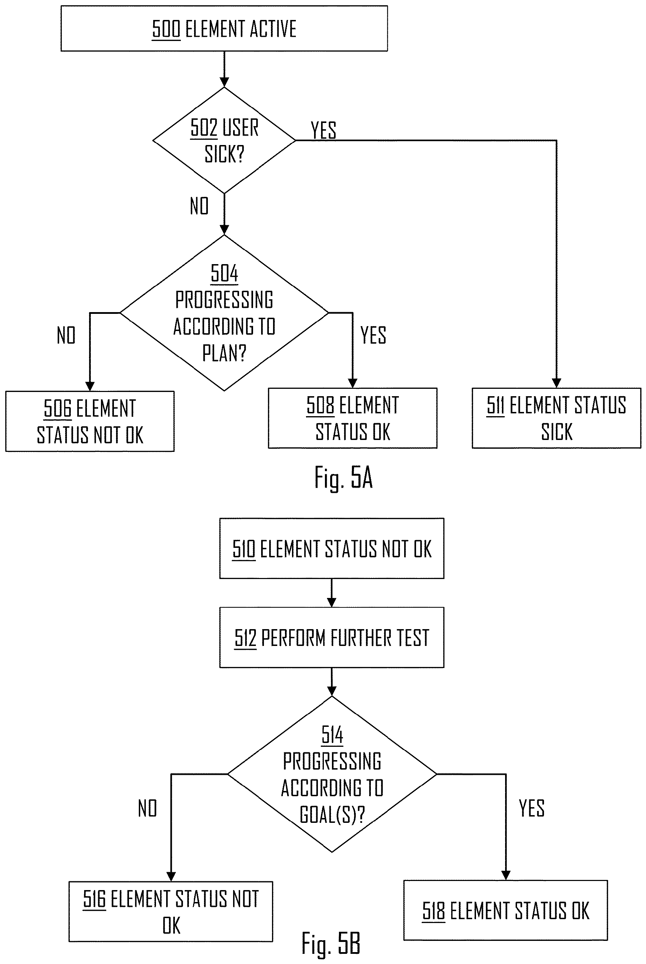

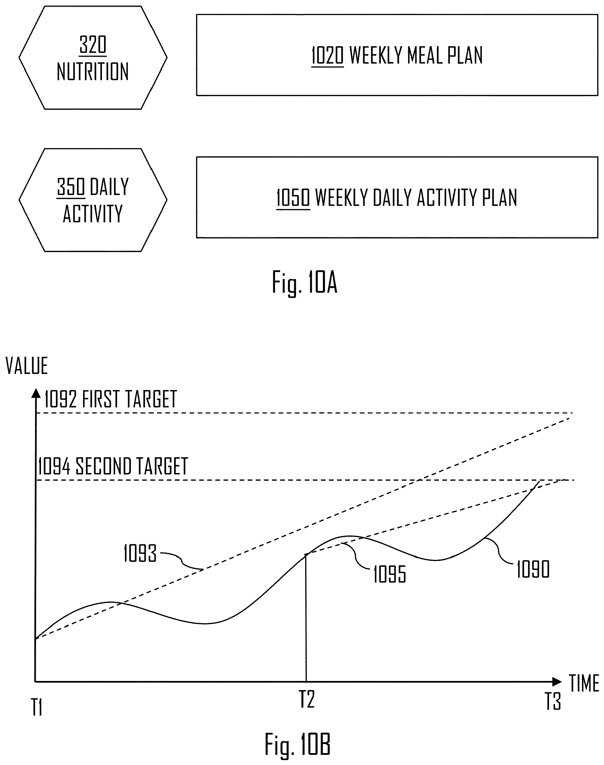

Figures 10A to 10C give examples according to some embodiments of how the activity plan may be generated and/or monitored. However, before proceeding on discussing the activity plan further, let us first look on some embodiments illustrated in Figures 5A to 6B.

Referring to Figure 5A, the computing device may determine that a coaching category is active (block 500). In block 502, the computing device may determine whether or not the user is sick or healthy. This determination may be based on user input and/or physiological measurement data. For example, cardiac activity data may be used reveal whether the user is sick or not. For example, the user account may comprise an algorithm for determining whether or not the user is sick or not. If the computing device determines that the user is sick, the computing device may then proceed to block 511 and set coaching category status to sick. In an embodiment, the computing device sets each active coaching category status to sick. In an embodiment, the computing device sets each coaching category status to sick. Thus, also inactive categories may be set to have sick status. The block 511 may be performed whenever the computing device determines that the user is sick, e.g. based on user input or physiological measurement data.

However, if in block 502 it is determined that the user is not sick, the computing device may proceed to block 504. In block 504, the computing device may determine whether or not the user is progressing according to the activity plan or according to the instructions. This determination may be coaching category -specific. Thus, the user may potentially be determined to proceed according to daily activity plan, but not according to meal plan. If the user is progressing according to the activity plan in the coaching category, the computing device may proceed to block 508. If not, the computing device may proceed to block 506. In block 506, the coaching category status may be set to not OK. In block 508, the coaching category status may be set to OK. It needs to be understood that, at least in some embodiments, the steps of Figure 5A may be performed to one or more coaching categories individually.

In an embodiment, the computing device is configured to obtain user data characterizing a physiological state of the user. The physiological state may mean the current state of the user or some previous state of the user. For example, one or more physiological tests, described above, may be used to determine the physiological state. Another example may be measured or inputted weight which may characterize the current weight of the user. For example, the user data may comprise user's height, gender, weight, and/or age. In an embodiment, the user data comprises data characterizing physical state of the user. For example, user's activity level may be indicated, such as low activity level, moderate activity level, and high activity level. One example, may comprise indication of a user's performance on a specific exercise (e.g. time on marathon, Cooper test result, swimming test result, and the like).

In an embodiment, the activity plan outputted to the user is based at least partly on the physiological state of the user and the one or more physiological goals of the user. In one example, a weight of the user may be measured to be 80 kilograms (kg). The user may have set a goal of getting more muscular. Further, time frame may be 8 weeks. Thus, the computing device may determine that 5 kg more weight may be a good goal for the body element 310, i.e. 85kg total weight after 8 weeks. Thus, the activity plan in the body element 310 may comprise instructions how to gain 5 kg during 8 weeks. For example, user may be instructed to eat enough calories and to eat right calories (e.g. protein, carbohydrates and fat in right mixture, and no alcohol). Similar logic applies to all coaching categories 300.

In an embodiment, the computing device is configured to monitor whether the user progresses according to the activity plan; and cause a performing of an action if the user is not progressing according to the activity plan. In an embodiment, the computing device causes a performing of a different action if the user is progressing according to the activity plan. In an embodiment, the progression is determined based further physiological measurement data of the user. For example, the system of Figure 1 may be configured to measure user's progression by performing further measurements. For example, weight may be measured during the timeframe of the activity plan.

In an embodiment, wherein the causing the performing of the action if the user is not progressing according to the activity plan comprises requiring a performing of at least one physiological test by the user (block 512 of Figure 5B). In an embodiment, the status of the coaching category is determined to be not OK (block 510). Block 512 may be performed as a response to the determination of block 510.

In block 514, the computing device may further determine, based on the results of the at least one physiological test (block 512), whether the user is progressing according to the activity plan. Based on the results, in an embodiment, the coaching category status may be set to OK (block 518) or not OK (block 516). For example, if meal stamps of the user indicate that the user is not progressing according to the activity plan for body element 310, the further test may be required by the computing device. Also, in an embodiment, the body element 310 is set to not OK state by the computing device. If the user performs the required test (e.g. weight measurement) and the result is according to the activity plan, the body element may be set to OK state. If the measurement indicates that the weight is not according to the activity plan, the element status may be set to not OK or maintained as not OK.

Another example may be related to marathon running. For example, if the user has set a goal of running a marathon, at least the training element 370 may comprise and output an activity plan suitable for reaching such goal. For example, the activity plan may comprise different length running exercises for a period of 8 weeks. The computing device may receive data from exercises performed by the user. If these exercises are according to the activity plan, the computing device may determine that the user is progressing according to the activity plan. However, if not, the computing device may require that a VO2MAX test needs to be performed. If the result of the test indicates that the user is actually progressing according to the activity plan (e.g. system may know which kind of VO2MAX result is needed to get through a marathon), the computing device may determine that the status of the training element 370 is OK.

In an embodiment, the computing device requires the at least one physiological test to be performed even though the user is progressing according to the activity plan. For example, a suitable test may be required to be performed once a week during an 8 week activity plan timeframe or period. The requiring may mean, for example, that the computing device does not provide further instructions if the user is not performing the at least one test with the system of Figure 1. However, in an embodiment, the user may skip such test by providing user input.

In an embodiment, the causing the performing of the action if the user is not progressing according to the activity plan comprises adapting the activity plan. For example, the timeframe or the period of the activity plan (e.g. lasts originally 8 weeks) may be made longer or shorter (e.g. 6 weeks, 10 weeks). Thus, the goal may be achieved in after a longer or shorter time. Another example of the adapting may be that the activity plan may be made more intensive or stricter. For example, the user may be required to perform additional exercises or lose weight faster.

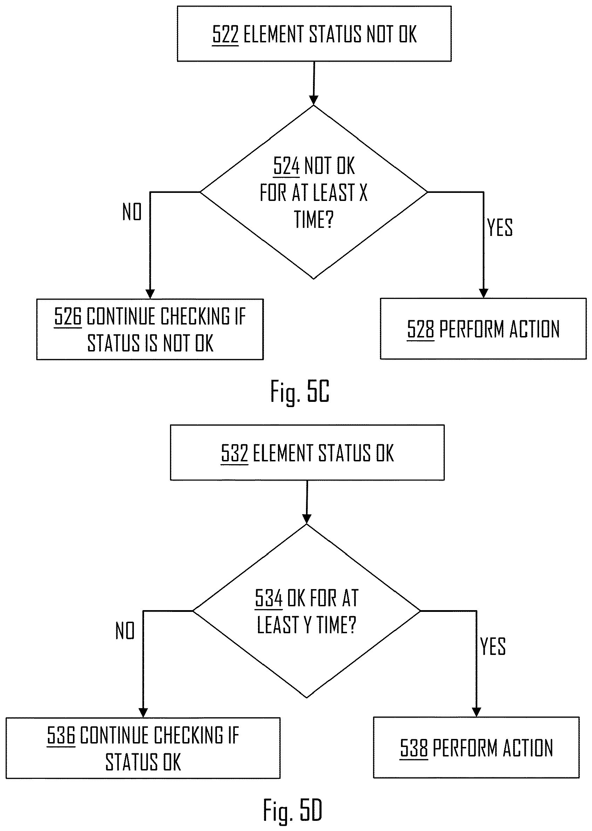

Referring to Figure 5C, in block 522 the computing device may determine that the status of a coaching category is not OK. In block 524, the computing device may determine whether the status has been not OK for at least or over a predetermined time. If yes, the computing device may perform an action in block 528. If the status has not been not OK for at least or over the predetermined time, the computing device may continue the checking process of blocks 522 and/or 524 (block 526). The action of block 528 may comprise, for example, adapting the action plan, requiring a physiological test to be performed and/or setting the coaching category state to inactive.

Similarly, referring to Figure 5D, in block 532 the computing device may determine that the status of a coaching category is OK. In block 534, the computing device may determine whether the status has been OK for at least or over a predetermined time. If yes, the computing device may perform an action in block 538. If the status has not been OK for at least or over the predetermined time, the computing device may continue the checking process of blocks 532 and/or 534 (block 536). The action of block 538 may comprise, for example, adapting the action plan to be more intense or stricter, and/or requesting a further physiological goal from the user. For example, the computing device may determine that the user can do better, based on the status being OK over a predetermined time, and thus require the user to set more ambitious goal(s).

At this point it needs to be discussed that the status of coaching elements may be at least "not OK" or "OK". Not OK -state may mean that the coaching element indicates that the user is not progressing according to the activity plan. OK -state may mean that the coaching element indicates that the user is progressing according to the activity plan. For example, the user interface of the computing device or a portable device used by the user may display the coaching categories. With one glance, the user may become aware in which coaching categories he/she is progressing according to the activity plan and in which he is not. This may make the training more efficient and/or focused on right area(s).

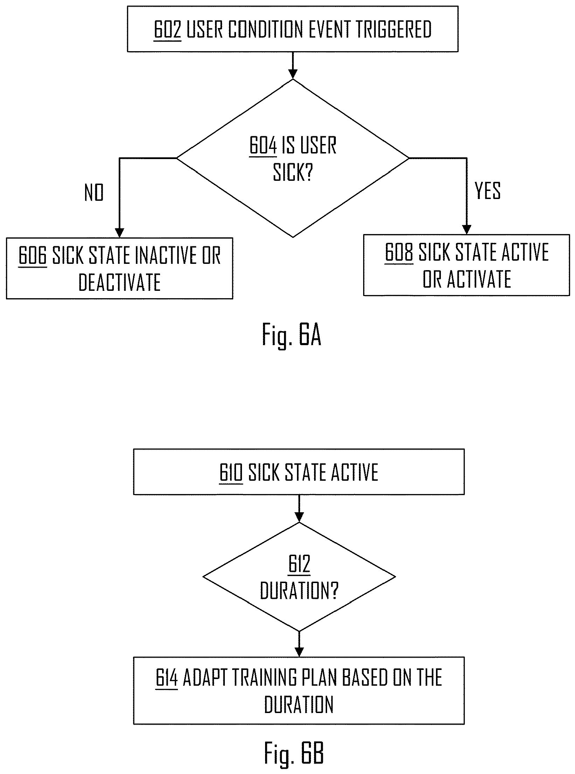

Figures 6A and 6B illustrate some embodiments in which, for example, user's sickness may be taken into account when determining and/or outputting the user instructions, such as the activity plan. Referring to Figure 6A, the computing device may determine that a user condition event is triggered (block 602), or the computing device may trigger the user event condition check. For example, user may provide input to his/her personal device that he/she is sick. Indication about this may be transmitted to the computing device (e.g. to the server 114). This may trigger the event of block 602. Further, the event may be triggered on to basis of measurement results (e.g. cardiac activity indicates sickness). Further, the event may be triggered if the physiological measurement data so indicates. For example, the physiological measurement data may indicate that the user has not been active for a predetermined time period (e.g. 3 days or 1 week). This may trigger the event. For example, if determined energy consumption, during the predetermined period, has not exceeded a threshold value, the computing device may determine that the user condition event should be triggered.

In block 604, the computing device may determine user's condition as a response to the user condition event triggering. For example, the computing device may request user input to indicate whether or not the user is sick. For example, if the user has not been active enough for the predetermined time (e.g. 3 days), the event may be triggered and the user may be asked to answer whether or not he/she feels sick or is sick. In another example, the computing device may cause performing of further measurements to determine whether or not the user is sick. For example, a physiological test may be requested to be performed by the user.

Based on the determination in block 604, the user account of the user may be set to healthy state or to sick state, as shown in blocks 606 and 608 respectively. For example, if the user account is in sick state, the computing device may be configured to prevent output of instructions to the user. In an embodiment, the computing device is configured to adapt the activity plan if the user account is in the sick state. Thus, the user's sickness may be taken into account by making the activity plan more realistic and/or not irritating the user with instructions when he/she is sick.

Referring to Figure 6B, one embodiment of adapting the activity plan is shown. If the computing device determines in block 610 that the user account is in sick state and/or that the user is sick, the computing device may further determine the length of sickness (block 612). In block 614, the computing device may adapt the activity plan based on the determined duration. For example, if the original activity plan was for a predetermined time (e.g. 8 weeks) and the user is sick for another predetermined time (e.g. 1 week), the activity plan may be adapted to last for a longer time period or the activity plan may be adapted to be stricter and/or more efficient (e.g. more weight loss in a shorter period or more exercises) during the same time period (i.e. 8 weeks).

Let us then discuss some embodiments with reference to Figures 10A to 10C. In an embodiment, the computing device is configured to determine whether the acquired physiological measurement data comprises data required for at least one algorithm of a first coaching category and data required for at least one algorithm of a second coaching category; and as a response to determining that the acquired physiological measurement data comprises the required data for the at least one algorithm of the first coaching category and the required data for the at least one algorithm of the second coaching category, the computing device is further configured to perform said algorithms, wherein the performing causes an output of coaching category -specific instructions to the user in the first coaching category and in the second coaching category. One example of this may be given in Figure 10A, wherein for the nutrition element 320 a weekly meal plan 1020 may be outputted and for the daily activity a weekly daily activity plan 1050 may be outputted. For example, the algorithms in each category may cause outputting of instructions and/or outputting of a value(s) characterizing physiological parameter of the user. For example, weight and meal plan may be outputted. For example, calorie consumption and a daily step goal may be outputted. These are examples of various different implementation possibilities.

The embodiment and the examples give above with respect to Figure 10A reveal one beneficial aspect. That is, using a plurality of coaching categories may enable giving specific instructions to user. Further, if at least two algorithms need to be performed before outputting the instructions (e.g. at least one algorithm in each of two coaching categories), the algorithms themselves may provide data that may be used to provide even more specific and accurate instructions. That is, the more there is measurement data available or the more algorithm are performed, the more accurate the activity plan may be.

Referring to Figure 10B, one example of adapting the activity plan may be shown. As discussed, the instructions for each category may comprise an activity plan indicating instructions for a predetermined time period and/or indicating one or more values characterizing state of the user (e.g. current weight, current activity level and the like). The computing device may first set a first target 1092 for the user in a coaching category. The first target 1092 may, for example, be some value (e.g. bench press result). The first activity plan 1093 may indicate and instruct how to get to the first target 1092. However, at time instance T2, the computing device may determine that the development 1090 of the user is not such that the first target 1092 may be achieved according to the first activity plan 1093. Thus, the computing device may determine a second target 1094 and a second activity plan 1095 to reach said target 1094. The second target 1094 may be more realistic for the user within the given time frame (e.g. T2-T3). In general, the computing device may adapt the activity plan for at least one of the coaching categories 300 based on physiological measurement data of the user.

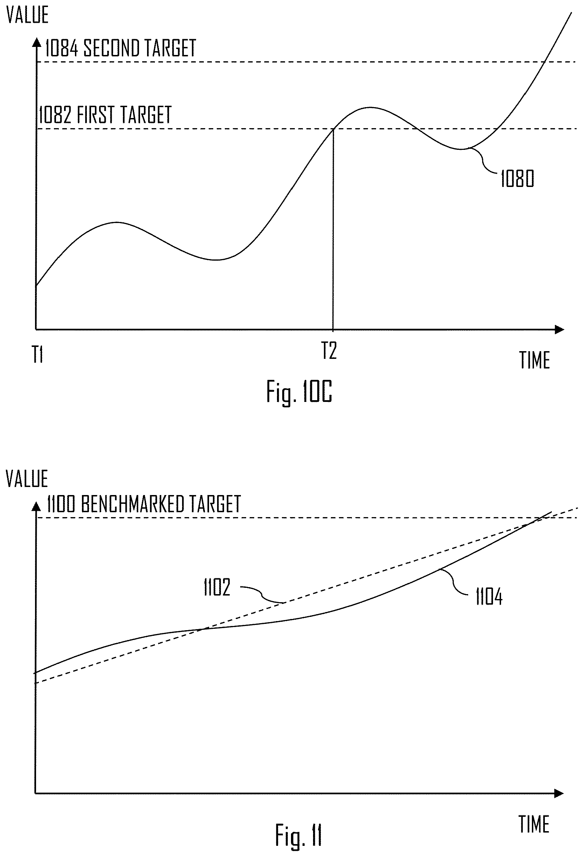

Referring to Figure 10C, another example of adapting the activity plan may be given. The computing device may determine that the user has reached the first target 1082 or will reach the first target 1082 (e.g. at time T2). The development may be indicated with the curve 1080. Thus, the computing device may determine and/or set a second target 1084 for the user. In the example of Figure 10C, the second target 1084 may be harder to achieve than the first target 1082. Thus, the computing device may cause the user to continue train hard by setting further and further targets in order for the user to reach his/her one or more physiological goals.

Figure 11 illustrates benchmarking to other users according to an embodiment. As discussed above, the computing device (e.g. the server 114) may manage a plurality of user accounts. Thus, in an embodiment, the computing device may determine and/or generate the activity plan for each coaching category based at least partly on performance of other users. For example, the computing device may determine how other users have achieved a weight loss target. For example, the computing device may associate the current user with a group of other users having similar user data (e.g. similar height, similar weight, same gender, same age, and the like). Then the computing device may benchmark the development of the current user to the development of the associated group. Thus, the activity plan for the current user may be further based on proven development of other users.

Referring to Figure 11, the computing device may set a benchmarked target 1100 to the user. For example, the target 1100 may be based on the associated group or at least some group of users (e.g. all users in the system of Figure 1). The computing device may determine an activity plan 1102 that is based on the benchmarked target 1100. Thus, the development of the user (e.g. curve 1104) may be generally thought to be more realistic when the benchmarking is used.

In an embodiment, the computing device is configured to cause outputting of coaching category -specific instructions in active coaching categories for a predetermined time without requiring a further algorithm to be performed. Thus, when at least one algorithm in a coaching category is performed, the instructions for that category may be outputted for the predetermined time. After the predetermined time, the outputting may be stopped.

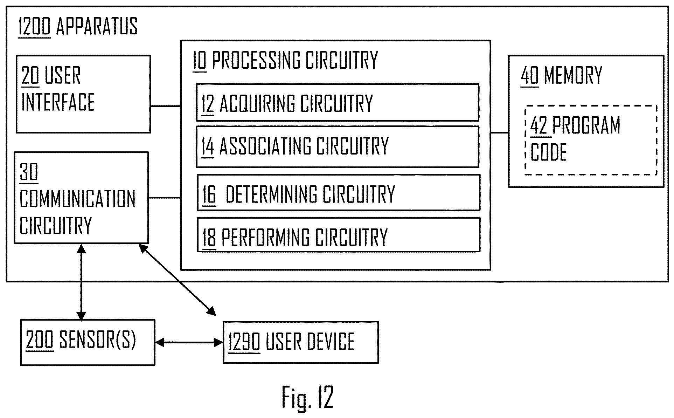

Figure 12 illustrates a block diagram of an apparatus according to an embodiment. The apparatus 1200 may be or be comprised in the computing device described above. The apparatus 1200 may be or be comprised in the apparatus performing the steps of Figure 8. In an embodiment, the apparatus 1200 is the server 114. In an embodiment, the apparatus 1200 is the wrist unit 102 or the PED 106.

The apparatus 1200 may comprise a user interface 20 for enabling user interaction with the apparatus 1200. For example, the user interface may comprise a display, a keypad, a speaker, and/or a microphone.

The apparatus 1200 may comprise a communication circuitry 30 configured to enable wireless and/or wired communication with external devices. For example, the communication circuitry 30 may comprise the wireless communication circuitry described above with respect to Figure 1. Thus, for example, Bluetooth, NFC, WLAN, LAN, 5kHz and/or cellular communication may be supported by the communication circuitry 30. Therefore, for example, the physiological measurement data may be received using the communication circuitry 30.

According to an embodiment, the apparatus 1200 carrying out the embodiments comprises a circuitry including at least one processor and at least one memory 40 including computer program code 42. When activated, the circuitry causes the apparatus to perform at least some of the functionalities according to any one of the embodiments, or operations thereof. For example, the method described in relation to Figure 8 may be performed by the circuitry.

In an embodiment, an apparatus carrying out the embodiments comprises means for performing the method according to any one of the embodiments, or operations thereof.

In an embodiment, there is provided a computer program product comprising program instructions which, when loaded into an apparatus, execute the method according to any one of the embodiments, or operations thereof. In an embodiment, there is provided a computer readable medium comprising said computer program.

In an embodiment, the apparatus comprises a processing circuitry 10 configured to perform operations of the apparatus 1200 according to any of the embodiments.

In an embodiment, the processing circuitry 10 comprises at least one processor.

In an embodiment, the processing circuitry 10 comprises an acquiring circuitry 12 configured to perform block 810 of Figure 8. The processing circuitry 10 may comprise an associating circuitry 14 configured to perform block 820 of Figure 8. The processing circuitry 10 may comprise a determining circuitry 16 configured to perform block 830 of Figure 8. The processing circuitry 10 may comprise a performing circuitry 18 configured to perform block 840 of Figure 8. The actions in each of the blocks 810, 820, 830, 840 should be understood as they are explained above with reference to Figure 8. Further, the circuitries 12-18 may be configured to perform additional actions of the apparatus 1200.

In an embodiment, the apparatus 1200 is the server 114. The server 114 may be connected to the sensor(s) 200 via the communication circuity 30. Further, the server 114 may be connected to a user device 1290 (e.g. wrist unit 102 or the PED 106) of the user.

In an embodiment, the apparatus 1200 is connected to the database 112. In an embodiment, the apparatus 1200 is connected to the network 110. In an embodiment, the memory 40 comprises the database 112 or at least part of the database 112.

In an embodiment, the apparatus 1200 is comprised in two or more physical entities. For example, the apparatus 1200 may comprise the server 114 and the database 112. The server 114 may be provided by a first physical entity and the database 112 by a second physical entity. In another example, some of the functionalities of the server 112 may be comprised in the first physical entity and some other functionalities in a second physical entity. Similar logic may apply to the database 112, for example.

In an embodiment, the algorithm(s) are referred to as algorithm(s) for processing physiological measurement data into at least one physiological value characterizing physiological parameter of the user. For example, energy consumption algorithm of daily activity element 350 may be configured to process motion data into a value indicating the energy consumption. Other examples of this processing should be apparent from the relevant data and acquired physiological values in each coaching category 300.

As used in this application, the term 'circuitry' refers to all of the following: (a) hardware-only circuit implementations, such as implementations in only analog and/or digital circuitry, and (b) combinations of circuits and software (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present. This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware. The term 'circuitry' would also cover, for example and if applicable to the particular element, a baseband integrated circuit or applications processor integrated circuit for a mobile phone or a similar integrated circuit in a server, a cellular network device, or another network device.

In an embodiment, at least some of the functionalities according to any one of the embodiments or operations thereof may be carried out by an apparatus comprising corresponding means for carrying out at least some of the described processes. Some example means for carrying out the processes may include at least one of the following: detector, processor (including dual-core and multiple-core processors), digital signal processor, controller, receiver, transmitter, encoder, decoder, memory, RAM, ROM, software, firmware, display, user interface, display circuitry, user interface circuitry, user interface software, display software, circuit, antenna, antenna circuitry, and circuitry. In an embodiment, the at least one processor, the memory, and the computer program code form processing means or comprises one or more computer program code portions for carrying out one or more operations according to any one of the embodiments or operations thereof.

The techniques and methods described herein may be implemented by various means. For example, these techniques may be implemented in hardware (one or more devices), firmware (one or more devices), software (one or more modules), or combinations thereof. For a hardware implementation, the apparatus(es) of embodiments may be implemented within one or more application-specific integrated circuits (ASICs), digital signal processors (DSPs), digital signal processing devices (DSPDs), programmable logic devices (PLDs), field programmable gate arrays (FPGAs), processors, controllers, microcontrollers, microprocessors, other electronic units designed to perform the functions described herein, or a combination thereof. For firmware or software, the implementation can be carried out through modules of at least one chip set (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, described with regard thereto, and they are not limited to the precise configurations set forth in the given figures, as will be appreciated by one skilled in the art.

Embodiments as described may also be carried out in the form of a computer process defined by a computer program. The computer program may be in source code form, object code form, or in some intermediate form, and it may be stored in some sort of carrier, which may be any entity or device capable of carrying the program. For example, the computer program may be stored on a computer program distribution medium readable by a computer or a processor. The distribution medium may be non-transitory and/or transitory, for example. The computer program medium may be, for example but not limited to, a record medium, computer memory, read-only memory, electrical carrier signal, telecommunications signal, and software distribution package, for example. Coding of software for carrying out the embodiments as shown and described is well within the scope of a person of ordinary skill in the art.

Even though the invention has been described above with reference to an example according to the accompanying drawings, it is clear that the invention is not restricted thereto but can be modified in several ways within the scope of the appended claims. Therefore, all words and expressions should be interpreted broadly and they are intended to illustrate, not to restrict, the embodiment. It will be obvious to a person skilled in the art that, as technology advances, the inventive concept can be implemented in various ways. Further, it is clear to a person skilled in the art that the described embodiments may, but are not required to, be combined with other embodiments in various ways.

## Claims

1. A computer-implemented method for physiological monitoring of a user, the method comprising:
acquiring (810), by an apparatus, physiological measurement data of the user, wherein the physiological measurement data was obtained by one or more sensor units;
associating (820), by the apparatus, the physiological measurement data with an user account of the user, wherein the user account comprises a plurality of physiological coaching categories (300) for the physiological monitoring of the user, each physiological coaching category comprising one or more algorithms configured to be performed by the apparatus and requiring physiological measurement data as an input and calculating physiological indicators for providing physiological coaching instructions;
determining (830), by the apparatus, whether the acquired physiological measurement data comprises data required by at least one of the algorithms; and
as a response to determining that the acquired physiological measurement data comprises the required data for the at least one algorithm, performing (840), by the apparatus, the at least one algorithm, wherein the performing causes an output of physiological coaching category -specific instructions (910-970) to the user in each of the coaching categories comprising the at least one performed algorithm, wherein a state of each of the plurality of coaching categories (300) is active or inactive, wherein only the algorithms of active coaching categories are configured to be performed, and in that the method further comprises:
activating (408) a coaching category if the acquired physiological measurement data comprises data required by at least one algorithm of said coaching category; and
determining whether or not a coaching category is active and causing output of said instructions only in active coaching categories, wherein said instructions (910-970) are not outputted in inactive coaching categories.

2. The method of claim 1, further comprising:
obtaining one or more physiological goals of the user, wherein the coaching category -specific instructions comprise an activity plan for reaching the one or more physiological goals.

3. The method of claim 2, further comprising:
obtaining user data characterizing a physiological state of the user, wherein the activity plan is based at least on the physiological state of the user and the one or more physiological goals of the user.

4. The method of any preceding claim 2 to 3, further comprising:
monitoring whether the user progresses according to the activity plan by acquiring further physiological measurement data of the user; and
causing a performing of an action if the user is not progressing according to the activity plan.

5. The method of claim 4, further comprising:
causing a performing of a different action if the user is progressing according to the activity plan.

6. The method of any preceding claim 4 to 5, wherein the causing the performing of the action if the user is not progressing according to the activity plan comprises requiring a performing _(512) of at least one physiological test by the user.

7. The method of claim 6, further comprising:
determining (514), based on the results of the at least one physiological test, whether the user is progressing according to the activity plan.

8. The method of any preceding claim 4 to 7, wherein the causing the performing of the action if the user is not progressing according to the activity plan comprises adapting the activity plan.

9. The method of any preceding claim 1 to 8, wherein the plurality of coaching categories (300) comprise at least two of the following coaching categories: a body parameter category (310), a nutrition category (320), a sleep category (330), a stress category (340), a daily activity category (350), a fitness category (360), and a training category (370).

10. The method of any preceding claim 1 to 9, wherein the measurement data comprises at least one of cardiac activity data, motion data, body weight data, body composition data, temperature data, blood pressure data, nutrition data, sleep quality data, sleep amount data, physiological test data.

11. The method of any preceding claim 1 to 10, wherein the one or more sensor units comprise at least one of electrode unit (202), optical sensor unit (204), bioimpedance sensor unit (206), temperature sensor unit (208), pressure sensor unit (210), blood pressure sensor unit (212), swimming sensor unit (214), cycling sensor unit (216), positioning sensor unit (218), motion sensor unit (220), weight scale (222).

12. The method of any preceding claim, further comprising:
activating or deactivating a coaching category according to a user input.

13. A system for physiological monitoring of a user, the system comprising:
a server computer (114) comprising at least one processor and at least one memory including a computer program code, wherein the at least one memory and the computer program code are configured, with the at least one processor, to cause the server computer (114) to perform operations comprising:
acquiring (810) physiological measurement data of a user, wherein the physiological measurement data was obtained by one or more sensor units;
associating (820) the physiological measurement data with an user account of the user, wherein the user account comprises a plurality of physiological coaching categories (300) for the physiological monitoring of the user, each physiological coaching category comprising one or more algorithms configured to be performed by the server computer and requiring physiological measurement data as an input and calculating physiological indicators for providing physiological coaching instructions;
determining (830) whether the acquired physiological measurement data comprises data required for at least one of the algorithms; and
as a response to determining that the acquired physiological measurement data comprises the required data for the at least one algorithm, performing (840) the at least one algorithm, wherein the performing causes an output of physiological coaching category -specific instructions (910-970) to the user in each of the coaching categories comprising the at least one performed algorithm;
wherein a state of each of the plurality of coaching categories is active or inactive, wherein only the algorithms of active coaching categories are configured to be performed, and in that the method further comprises:
activating (408) a coaching category if the acquired physiological measurement data comprises data required by at least one algorithm of said coaching category; and
determining whether or not a coaching category is active and causing output of said instructions only in active coaching categories, wherein said instructions (910-970) are not outputted in inactive coaching categories.

14. A computer program product embodied on a distribution medium readable by a data processing system and comprising program instructions which, when executed by a data processing system, cause the data processing system to execute the method according to any of claims 1 to 12.

## Patentansprüche

1. Computerimplementiertes Verfahren zum physiologischen Überwachen eines Benutzers, wobei das Verfahren Folgendes umfasst:
Erfassen (810) von physiologischen Messdaten des Benutzers durch eine Vorrichtung, wobei die physiologischen Messdaten von einer oder mehreren Sensoreinheiten erhalten wurden;
Verknüpfen (820) der physiologischen Messdaten durch die Vorrichtung mit einem Benutzerkonto des Benutzers, wobei das Benutzerkonto eine Vielzahl von physiologischen Coachingkategorien (300) für die physiologische Überwachung des Benutzers umfasst, wobei jede physiologische Coachingkategorie einen oder mehrere Algorithmen umfasst, die dazu ausgelegt sind, von der Vorrichtung durchgeführt zu werden, und physiologische Messdaten als eine Eingabe erfordert und physiologische Indikatoren zum Bereitstellen von physiologischen Coachinganweisungen berechnet;
Bestimmen (830) durch die Vorrichtung, ob die erfassten physiologischen Messdaten Daten umfassen, die für mindestens einen der Algorithmen erforderlich sind; und
als Reaktion auf das Bestimmen, dass die erfassten physiologischen Messdaten die erforderlichen Daten für den mindestens einen Algorithmus umfassen, Durchführen (840) des mindestens einen Algorithmus durch die Vorrichtung, wobei das Durchführen einer Ausgabe von physiologischen coachingkategoriespezifischen Anweisungen (910-970) für den Benutzer in jeder der Coachingkategorien, die den mindestens einen durchgeführten Algorithmus umfassen, bewirkt, wobei ein Zustand von jeder der Vielzahl von Coachingkategorien (300) aktiv oder inaktiv ist, wobei nur die Algorithmen von aktiven Coachingkategorien dazu ausgelegt sind, durchgeführt zu werden, und dadurch, dass das Verfahren ferner Folgendes umfasst:
Aktivieren (408) einer Coachingkategorie, wenn die erfassten physiologischen Messdaten Daten umfassen, die für mindestens einen Algorithmus der Coachingkategorie erforderlich sind; und
Bestimmen, ob eine Coachingkategorie aktiv ist oder nicht, und Bewirken einer Ausgabe der Anweisungen nur in aktiven Coachingkategorien, wobei die Anweisungen (910-970) in inaktiven Coachingkategorien nicht ausgegeben werden.

2. Verfahren nach Anspruch 1, das ferner Folgendes umfasst:
Erhalten von einem oder mehreren physiologischen Zielen des Benutzers, wobei die coachingkategoriespezifischen Anweisungen einen Aktivitätsplan zum Erreichen des einen oder der mehreren physiologischen Ziele umfassen.

3. Verfahren nach Anspruch 2, das ferner Folgendes umfasst:
Erhalten von Benutzerdaten, die einen physiologischen Zustand des Benutzers charakterisieren, wobei der Aktivitätsplan mindestens auf dem physiologischen Zustand des Benutzers und dem einen oder den mehreren physiologischen Zielen des Benutzers basiert.

4. Verfahren nach einem der vorhergehenden Ansprüche 2 bis 3, das ferner Folgendes umfasst:
Überwachen, ob der Benutzer gemäß dem Aktivitätsplan fortschreitet, durch Erfassen von weiteren physiologischen Messdaten des Benutzers; und
Bewirken eines Durchführens einer Aktion, wenn der Benutzer nicht gemäß dem Aktivitätsplan fortschreitet.

5. Verfahren nach Anspruch 4, das ferner Folgendes umfasst:
Bewirken eines Durchführens einer anderen Aktion, wenn der Benutzer gemäß dem Aktivitätsplan fortschreitet.

6. Verfahren nach einem der vorhergehenden Ansprüche 4 bis 5, wobei das Bewirken des Durchführens der Aktion, wenn der Benutzer nicht gemäß dem Aktivitätsplan fortschreitet, das Erfordern eines Durchführen (512) von mindestens einem physiologischen Test durch den Benutzer umfasst.

7. Verfahren nach Anspruch 6, das ferner Folgendes umfasst:
Bestimmen (514) auf Basis der Ergebnisse des mindestens einen physiologischen Tests, ob der Benutzer gemäß dem Aktivitätsplan fortschreitet.

8. Verfahren nach einem der vorhergehenden Ansprüche 4 bis 7, wobei das Bewirken des Durchführens der Aktion, wenn der Benutzer nicht gemäß dem Aktivitätsplan fortschreitet, das Anpassen des Aktivitätsplans umfasst.

9. Verfahren nach einem der vorhergehenden Ansprüche 1 bis 8, wobei die Vielzahl von Coachingkategorien (300) mindestens zwei der folgenden Coachingkategorien umfassen: eine Körperparameterkategorie (310), eine Ernährungskategorie (320), eine Schlafkategorie (330), eine Belastungskategorie (340), eine tägliche Aktivitätskategorie (350), eine Fitnesskategorie (360) und eine Trainingskategorie (370).

10. Verfahren nach einem der vorhergehenden Ansprüche 1 bis 9, wobei die Messdaten mindestens eines von Herzaktivitätsdaten, Bewegungsdaten, Körpergewichtsdaten, Körperzusammensetzungsdaten, Temperaturdaten, Blutdruckdaten, Ernährungsdaten, Schlafqualitätsdaten, Schlafmengendaten, physiologischen Testdaten umfassen.

11. Verfahren nach einem der vorhergehenden Ansprüche 1 bis 10, wobei die eine oder die mehreren Sensoreinheiten mindestens eines von einer Elektrodeneinheit (202), einer optischen Sensoreinheit (204), einer Bioimpedanzsensoreinheit (206), einer Temperatursensoreinheit (208), einer Drucksensoreinheit (210), einer Blutdrucksensoreinheit (212), einer Schwimmsensoreinheit (214), einer Radfahrsensoreinheit (216), einer Positionierungssensoreinheit (218), einer Bewegungssensoreinheit (220), einer Gewichtsskala (222) umfassen.

12. Verfahren nach einem der vorhergehenden Ansprüche, das ferner Folgendes umfasst:
Aktivieren oder Deaktivieren einer Coachingkategorie gemäß einer Benutzereingabe.

13. System zum physiologischen überwachen eines Benutzers, wobei das System Folgendes umfasst:
einen Servercomputer (114), der mindestens einen Prozessor und mindestens einen Speicher umfasst, der einen Computerprogrammcode beinhaltet, wobei der mindestens eine Speicher und der Computerprogrammcode mit dem mindestens einen Prozessor dazu ausgelegt sind zu bewirken, dass der Servercomputer (114) die folgenden Operationen durchführt:
Erfassen (810) von physiologischen Messdaten eines Benutzers, wobei die physiologischen Messdaten von einer oder mehreren Sensoreinheiten erhalten wurden;
Verknüpfen (820) der physiologischen Messdaten mit einem Benutzerkonto des Benutzers, wobei das Benutzerkonto eine Vielzahl von physiologischen Coachingkategorien (300) für die physiologische Überwachung des Benutzers umfasst, wobei jede physiologische Coachingkategorie einen oder mehrere Algorithmen umfasst, die dazu ausgelegt sind, vom Servercomputer durchgeführt zu werden, und physiologische Messdaten als eine Eingabe erfordert und physiologische Indikatoren zum Bereitstellen von physiologischen Coachinganweisungen berechnet;
Bestimmen (830), ob die erfassten physiologischen Messdaten Daten umfassen, die für mindestens einen der Algorithmen erforderlich sind; und
als Reaktion auf das Bestimmen, dass die erfassten physiologischen Messdaten die erforderlichen Daten für den mindestens einen Algorithmus umfassen, Durchführen (840) des mindestens einen Algorithmus, wobei das Durchführen einer Ausgabe von physiologischen coachingkategoriespezifischen Anweisungen (910-970) für den Benutzer in jeder der Coachingkategorien, die den mindestens einen durchgeführten Algorithmus umfassen, bewirkt;
wobei ein Zustand von jeder der Vielzahl von Coachingkategorien aktiv oder inaktiv ist, wobei nur die Algorithmen von aktiven Coachingkategorien dazu ausgelegt sind, durchgeführt zu werden, und dadurch, dass das Verfahren ferner Folgendes umfasst:
Aktivieren (408) einer Coachingkategorie, wenn die erfassten physiologischen Messdaten Daten umfassen, die für mindestens einen Algorithmus der Coachingkategorie erforderlich sind; und
Bestimmen, ob eine Coachingkategorie aktiv ist oder nicht, und Bewirken einer Ausgabe der Anweisungen nur in aktiven Coachingkategorien, wobei die Anweisungen (910-970) in inaktiven Coachingkategorien nicht ausgegeben werden.

14. Computerprogrammprodukt, das auf einem Verteilungsmedium enthalten ist, das von einem Datenverarbeitungssystem lesbar ist und Programmanweisungen umfasst, die, wenn sie von einem Datenverarbeitungssystem ausgeführt werden, bewirken, dass das Datenverarbeitungssystem das Verfahren nach einem der Ansprüche 1 bis 12 ausführt.

## Revendications

1. Procédé mis en œuvre par ordinateur pour une surveillance physiologique d'un utilisateur, le procédé comprenant :
l'acquisition (810), par un appareil, de données de mesure physiologique de l'utilisateur, où les données de mesure physiologique ont été obtenues par une ou plusieurs unités de capteur ;
l'association (820), par l'appareil, des données de mesure physiologique à un compte utilisateur de l'utilisateur, où le compte utilisateur comprend une pluralité de catégories de coaching physiologique (300) pour la surveillance physiologique de l'utilisateur, chaque catégorie de coaching physiologique comprenant un ou plusieurs algorithmes configurés pour être exécutés par l'appareil et requérant des données de mesure physiologique en entrée et calculant des indicateurs physiologiques pour fournir des instructions de coaching physiologique ;
la détermination (830), par l'appareil, de si les données de mesure physiologique acquises comprennent des données requises par au moins l'un des algorithmes ; et
en réponse à la détermination que les données de mesure physiologique acquises comprennent les données requises pour le au moins un algorithme, l'exécution (840), par l'appareil, du au moins un algorithme, où l'exécution provoque une sortie d'instructions spécifiques à une catégorie de coaching physiologique (910-970) vers l'utilisateur dans chacune des catégories de coaching comprenant le au moins un algorithme exécuté, dans lequel un état de chacune de la pluralité de catégories de coaching (300) est actif ou inactif, où seuls les algorithmes de catégories de coaching actives sont configurés pour être exécutés, et en ce que le procédé comprend en outre :
l'activation (408) d'une catégorie de coaching si les données de mesure physiologique acquises comprennent des données requises par au moins un algorithme de ladite catégorie de coaching ; et
la détermination de si oui ou non une catégorie de coaching est active et le fait de provoquer la sortie desdites instructions uniquement dans des catégories de coaching actives, où lesdites instructions (910-970) ne sont pas sorties dans des catégories de coaching inactives.

2. Procédé selon la revendication 1, comprenant en outre :
l'obtention d'un ou plusieurs objectifs physiologiques de l'utilisateur, où les instructions spécifiques à une catégorie de coaching comprennent un plan d'activité pour atteindre les un ou plusieurs objectifs physiologiques.

3. Procédé selon la revendication 2, comprenant en outre :
l'obtention de données d'utilisateur caractérisant un état physiologique de l'utilisateur, où le plan d'activité est basé au moins sur l'état physiologique de l'utilisateur et les un ou plusieurs objectifs physiologiques de l'utilisateur.

4. Procédé selon l'une quelconque des revendications 2 à 3 précédentes, comprenant en outre :
la surveillance de si l'utilisateur progresse conformément au plan d'activité en acquérant des données de mesure physiologique supplémentaires de l'utilisateur ; et
le fait de provoquer une exécution d'une action si l'utilisateur ne progresse pas conformément au plan d'activité.

5. Procédé selon la revendication 4, comprenant en outre :
le fait de provoquer une exécution d'une action différente si l'utilisateur progresse conformément au plan d'activité.

6. Procédé selon l'une quelconque des revendications 4 à 5 précédentes, dans lequel le fait de provoquer l'exécution de l'action si l'utilisateur ne progresse pas conformément au plan d'activité comprend le fait de requérir une exécution (512) d'au moins un test physiologique par l'utilisateur.

7. Procédé selon la revendication 6, comprenant en outre :
la détermination (514), sur la base des résultats du au moins un test physiologique, de si l'utilisateur progresse conformément au plan d'activité.

8. Procédé selon l'une quelconque des revendications 4 à 7 précédentes, dans lequel le fait de provoquer l'exécution de l'action si l'utilisateur ne progresse pas conformément au plan d'activité comprend l'adaptation du plan d'activité.

9. Procédé selon l'une quelconque des revendications 1 à 8 précédentes, dans lequel la pluralité de catégories de coaching (300) comprend au moins deux des catégories de coaching suivantes : une catégorie de paramètre corporel (310), une catégorie de nutrition (320), une catégorie de sommeil (330), une catégorie de stress (340), une catégorie d'activité quotidienne (350), une catégorie de forme physique (360), et une catégorie d'entraînement (370).

10. Procédé selon l'une quelconque des revendications 1 à 9 précédentes, dans lequel les données de mesure comprennent au moins l'une parmi des données d'activité cardiaque, des données de mouvement, des données de poids corporel, des données de composition corporelle, des données de température, des données de pression artérielle, des données de nutrition, des données de qualité de sommeil, des données de quantité de sommeil, des données de test physiologique.

11. Procédé selon l'une quelconque des revendications 1 à 10 précédentes, dans lequel les une ou plusieurs unités de capteur comprennent au moins l'une parmi une unité d'électrode (202), une unité de capteur optique (204), une unité de capteur de bioimpédance (206), une unité de capteur de température (208), une unité de capteur de pression (210), une unité de capteur de pression artérielle (212), une unité de capteur de nage (214), une unité de capteur de pédalage (216), unité de capteur de positionnement (218), une unité de capteur de mouvement (220), une échelle de poids (222).

12. Procédé selon l'une quelconque des revendications précédentes, comprenant en outre :
l'activation ou la désactivation d'une catégorie de coaching conformément à une entrée d'utilisateur.

13. Système pour une surveillance physiologique d'un utilisateur, le système comprenant :
un ordinateur serveur (114) comprenant au moins un processeur et au moins une mémoire incluant un code de programme informatique, où la au moins une mémoire et le code de programme informatique sont configurés, avec le au moins un processeur, pour amener l'ordinateur serveur (114) à effectuer des opérations comprenant :
l'acquisition (810) de données de mesure physiologique d'un utilisateur, où les données de mesure physiologique ont été obtenues par une ou plusieurs unités de capteur ;
l'association (820) des données de mesure physiologique à un compte utilisateur de l'utilisateur, où le compte utilisateur comprend une pluralité de catégories de coaching physiologique (300) pour la surveillance physiologique de l'utilisateur, chaque catégorie de coaching physiologique comprenant un ou plusieurs algorithmes configurés pour être exécutés par l'ordinateur serveur et requérant des données de mesure physiologique en entrée et calculant des indicateurs physiologiques pour fournir des instructions de coaching physiologique ;
la détermination (830) de si les données de mesure physiologique acquises comprennent des données requises pour au moins l'un des algorithmes ; et
en réponse à la détermination que les données de mesure physiologique acquises comprennent les données requises pour le au moins un algorithme, l'exécution (840) du au moins un algorithme, où l'exécution provoque une sortie d'instructions spécifiques à une catégorie de coaching physiologique (910-970) vers l'utilisateur dans chacune des catégories de coaching comprenant le au moins un algorithme exécuté ;
dans lequel un état de chacune de la pluralité de catégories de coaching est actif ou inactif, où seuls les algorithmes de catégories de coaching actives sont configurés pour être exécutés, et en ce que le procédé comprend en outre :
l'activation (408) d'une catégorie de coaching si les données de mesure physiologique acquises comprennent des données requises par au moins un algorithme de ladite catégorie de coaching ; et
la détermination de si oui ou non une catégorie de coaching est active et le fait de provoquer la sortie desdites instructions uniquement dans des catégories de coaching actives, où lesdites instructions (910-970) ne sont pas sorties dans des catégories de coaching inactives.

14. Produit de programme informatique incorporé sur un support de distribution lisible par un système de traitement de données et comprenant des instructions de programme qui, lorsqu'elles sont exécutées par un système de traitement de données, amènent le système de traitement de données à exécuter le procédé selon l'une quelconque des revendications 1 à 12.
